# EUROPEAN PATENT SPECIFICATION

(11) **EP 1 957 493 B1**
(45) Date of publication and mention of the grant of the patent: **19.01.2011**
(21) Application number: 06830185.2
(22) Date of filing: 29.11.2006
(51) Int. Cl.: C07D 451/06

(54) **8-AZABICYCLO[3.2.1]OCTANE DERIVATIVES USEFUL AS MONOAMINE REUPTAKE INHIBITORS**
ALS MONOAMIN WIEDERAUFNAHMEHEMMER GEEIGNETE 8-AZABICYCLO[3.2.1]OCTANDERIVATE
DERIVES DE 8-AZABICYCLO[3.2.1]OCTANE UTILES EN TANT QU'INHIBITEURS DU RECAPTAGE DE LA MONOAMINE

(30) Priority: 01.12.2005 EP 05111578
(43) Date of publication of application: 20.08.2008
(73) Proprietor: N.V. Organon, 5349 AB Oss (NL)
(72) Inventor: NAPIER, Susan Elizabeth, Newhouse Central Scotland ML 1 5SH (GB); BINGHAM, Matilda, Jane, Newhouse Central Scotland ML1 5SH (GB); DUNBAR, Neil, Andrew, Newhouse Central Scotland ML1 5SH (GB)
(74) Representative: van Wezenbeek, Petrus M.G.F.
(86) International application number: PCT/EP2006/069047
(87) International publication number: WO 2007/063071

(56) References cited:
- WO-A-2004/113334
- GB-A- 1 164 555
- ANANTHAN, SUBRAMANIAM ET AL: "Identification of a novel partial inhibitor of dopamine transporter among 4-substituted 2-phenylquinazolines" BIOORGANIC & MEDICINAL CHEMISTRY LETTERS , 12(16), 2225-2228 CODEN: BMCLE8; ISSN: 0960-894X, 2002, XP002395822 cited in the application

## Description

The present invention relates to 8-azabicyclo[3.2.1]octane derivatives, to pharmaceutical compositions comprising these compounds and to their use in therapy.

Monoamine reuptake inhibitors have found widespread use in therapy, in particular, in the treatment of depression, a common, serious and life-threatening disorder with persistent and debilitating side-effects. The older tricyclic monoamine reuptake inhibitors, including imipramine and amitriptyline are effective antidepressants but these compounds additionally have deleterious cardiovascular and anticholinergic side-effects which can lead to serious toxicity in overdose and to poor patient compliance. The newer drugs, such as the selective serotonin reuptake inhibitors (SSRls), whilst being an improvement over older antidepressants have their own particular pattern of side-effects which include sleep disturbances, gastrointestinal symptoms and sexual problems. Monoamine reuptake inhibitors are also indicated to be useful in the treatment of other disorders such as pain, panic disorders, depression, anxiety, attention deficit hyperactivity disorder (ADHD) or obsessive compulsive disorder.

In view of the shortcomings of the currently available monoamine reuptake inhibitors, the search for new compounds which are safe and effective continues. In particular, there has been recently renewed interest in drugs which inhibit monoamine reuptake at one or more of the serotonin, noradrenaline and dopamine transporters.

WO 04/113334 discloses 8-azabicyclo[3.2.1]octane derivatives indicated to be monoamine neurotransmitter reuptake inhibitors and as such useful in the treatment of diseases or disorders responsive to inhibition of monoamine neurotransmitter reuptake in the central nervous system.

A further example of a 8-azabicyclo[3.2.1]octane derivative, shown to bind weakly to serotonin and dopamine transporters is disclosed in Bioorg. and Med. Chem. Lett., 2002, 12, 2225-2228. The 2-phenylquinazoline group is a key feature of all the dopamine transporter ligands disclosed. The 8-azabicyclo[3.2.1]octane derivative disclosed is not indicated to inhibit reuptake of either serotonin or dopamine.

GB 1164555 discloses tropine derivatives, including 8-alkyl-3-aryloxy-8-azabicyclo[3.2.1 ]octanes indicated to possess effective and unexpected pharmacological properties. 8-Methyl-3-(2-phenylphenoxy)-8-azabicyclo[3.2.1]octane is specifically disclosed.

In a first aspect the present invention provides a 8-azabicyclo[3.2.1]octane derivative of formula I 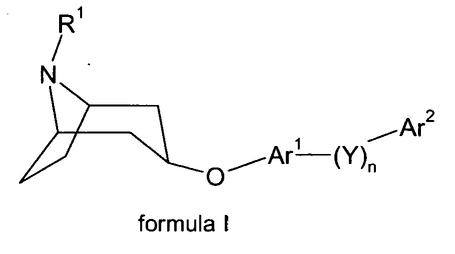 wherein
R¹ is H or C₁₋₅alkyl;
Y is O, S or O(CH₂)ₘ;
m is 1 or 2;
n is 0 or 1;
Ar¹ is phenylene or pyridylene, said phenylene and pyridylene being 1,3-linked with respect to O and when n is 1 with Y and when n is 0 with Ar², said phenylene or pyridylene being optionally substituted with one or two substituents independently selected from halogen, C₁₋₅alkyl, C₁₋₅alkoxy, C₃₋₆cycloalkyl, C₂₋₅alkenyl, C₂₋₅alkynyl, phenyl, CN and hydroxy, wherein said C₁₋₅alkyl and C₁₋₅alkoxy are optionally substituted with one to three halogens and wherein the oxygen of said hydroxy is optionally bonded to Ar² to form a 5-membered ring;
Ar² is phenyl or a 5-6 membered heteroaryl, said phenyl or 5-6 membered heteroaryl being optionally substituted with one to three substituents independently selected from halogen, C₁₋₅alkyl, C₁₋₅alkoxy, CN, CONR²R³, CO₂R⁴, NHCOR⁵ and hydroxy, wherein said C₁₋₅alkyl and C₁₋₅alkoxy are optionally substituted with one to three halogens and wherein the oxygen of said hydroxy is optionally bonded to Ar¹ to form a 5-membered ring;
R²-R⁴ are independently H or C₁₋₅alkyl and
R⁵ is C₁₋₅alkyl
or a pharmaceutically acceptable salt or solvate thereof.

The term C₁₋₅alkyl, as used herein, represents a branched or unbranched alkyl group having 1-5 carbon atoms. Examples of such groups are methyl, ethyl, isopropyl, tertiary butyl and pentyl.

The term C₁₋₅alkoxy, as used herein, represents a branched or unbranched alkoxy group having 1-5 carbon atoms. Examples of such groups are methoxy, ethoxy, isopropyloxy and tertiary butyloxy.

The term C₃₋₆cycloalkyl, as used herein, represents a branched or unbranched cyclic alkyl group having 3-6 carbon atoms. Examples of such groups are cyclopropyl, cyclopentyl and 2-methylcyclopentyl.

The term C₂₋₅alkenyl, as used herein, represents a branched or unbranched alkenyl group having 2-5 carbon atoms and at least one double bond. Examples of such groups are ethenyl and propenyl.

The term C₂₋₅ alkynyl, as used herein, represents a branched or unbranched alkynyl group having 2-5 carbon atoms and at least one triple bond. Examples of such groups are ethynyl and propynyl.

The term 5-6 membered heteroaryl ring, as used herein, represents a 5-6 membered heteroaromatic ring comprising 1-2 heteroatoms selected from N, O and S. Examples of such groups include furanyl, pyrrolyl, thienyl, pyridinyl, oxazolyl, imidazolyl, thiazolyl and pyrimidinyl.

The term halogen, as used herein, represents a F, Cl, Br or I atom

The skilled person will appreciate that when Ar¹ is pyridylene 1,3-linked with respect to O and when n is 1 with Y and when n is 0 with Ar², the term '1,3-tinkled' refers to the linking relationship of the pyridylene with respect to O and when n is 1 with Y and when n is 0 with Ar² and not with respect to the numbering of the pyridylene. Thus the skilled person will appreciate that with respect to the numbering of the pyridylene Ar¹ is, for example, a 2,6-pyridylene or a 2,4-pyridylene but not a 1,3-pyridylene.

In one embodiment of the present invention R¹ is H.

In a further embodiment R¹ is methyl.

In a further embodiment n is 0.

In another embodiment Y is O and n is 1.

In another embodiment Ar¹ is phenylene or pyridylene optionally substituted with one or two substituents independently selected from halogen, C₁₋₅alkyl, C₁₋₅alkoxy, or CN. In a further embodiment Ar¹ is phenylene or pyridylene optionally substituted with one or two substituents independently selected from chloro, fluoro, methyl, methoxy or CN.

A further embodiment of the present invention is a 8-azabicyclo[3.2.1]octane derivative of formula II 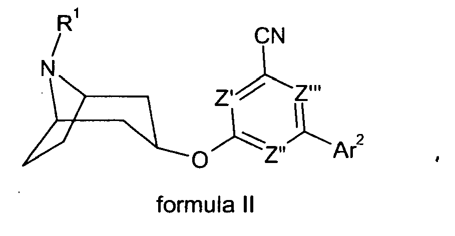 wherein Z', Z" and Z'" are CH or N with the proviso that only one of Z', Z" and Z'" can be N at the same time and wherein R¹ and Ar² have the previously defined meanings. A still further embodiment of the present invention is a 8-azabicyclo[3.2.1]octane derivative of formula II, wherein Z' and Z'" are CH, Z" is CH or N and wherein R¹ and Ar² have the previously defined meanings.

In a further embodiment Ar² is phenyl or a 5-6 membered heteroaryl, said phenyl or 5-6 membered heteroaryl being optionally substituted with one or two substituents independently selected from halogen, C₁₋₅alkyl, C₁₋₅alkoxy and CN wherein said C₁₋₅alkyl is optionally substituted with 1-3 halogens. In a still further embodiment Ar² is phenyl or pyridyl said phenyl or pyridyl being optionally substituted with one to two substituents independently selected from chloro, fluoro, methyl, methoxy, CN or CF₃.

A further embodiment of the present invention is a 8-azabicyclo[3.2.1]octane derivative of formula III 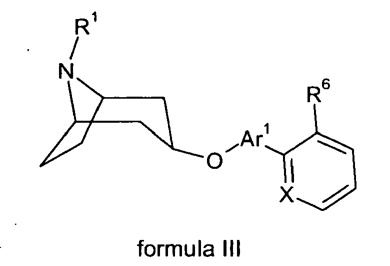 wherein R¹ and Ar¹ have the previously defined meanings and wherein X is CH or N and R⁶ is H, methoxy, fluoro, chloro, CN or CF₃.

A still further embodiment of the present invention is a 8-azabicyclo[3.2.1]octane derivative of formula IV 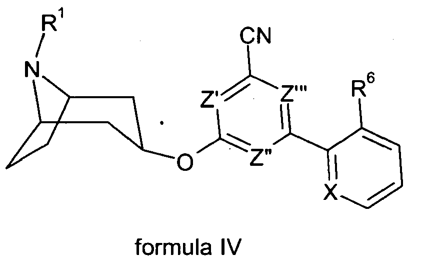 wherein R¹ has the previously defined meanings, Z', Z" and Z"' are CH or N with the proviso that only one of Z', Z" and Z'" can be N at the same time and wherein X is CH or N and R⁶ is H, methoxy, fluoro, chloro, CN or CF₃.

A further embodiment of the present invention is a 8-azabicyclo[3.2.1]octane derivative selected from:
3-*exo*-(5-chlorobiphenyl-3-yloxy)-8-azabicyclo[3.2.1]octane;
*exo* 5-(8-azabicyclo[3.2.1]oct-3-yloxy)biphenyl-3-carbonitrile;
*exo* 3-(8-azabicyclo[3.2.1]oct-3-yloxy)-5-phenoxybenzonitrile;
3-*exo*-(4'-methoxybiphenyl-3-yloxy)-8-azabicyclo[3.2.1.]octane;
*exo* 3'-(8-azabicyclo[3.2.1]oct-3 -yloxy)biphenyl-4-carbonitrile;
3-*exo*-(3-pyridin-4-ylphenoxy)-8-azabicyclo[3.2.1]octane;
*exo* 2-{6-(8-azabicyclo[3.2.1]oct-3-yloxy)-4-chloropyridin-2-yl}benzonitrile;
*exo* 2-(8-azabicyclo[3.2.1]oct-3-yloxyr6-(2-cyanophenyl)isonicotinonitrile;
*exo* 3-[(8-azabicyclo[3.2.1]oct-3-yl)oxy]-5-(3-chloropyridin-2-yl)benzonitrile;
*exo* 3-(8-azabicyclo[3.2.1]oct-3-yloxy)-5-cyanophenyl)nicotinonitrile;
*exo* 3-(8-azabicyclo[3.2.1]oct-3-yloxy)-5-(3-fluoropyridin-2-yl)benzonitrile;
*exo* 3'-(8-azabicyclo[3.2.1]oct-3-yloxy)biphenyl-2-carbonitrile;
*exo* 2-[6-(8-azabicyclo[3.2.1]oct-3-yloxy)pyridin-2-yl]benzonitrile;
3'-(8-azabicyclo[3.2.1]oct-3-*exo*-yloxy)-2'-fluorobiphenyl-4-carbonitrile and
2-(8-azabicyclo[3.2.1]oct-3-*exo*-yloxy)-6-phenylisonicotinonitrile
or a pharmaceutically acceptable salt or solvate thereof.

The 8-azabicyclo[3.2.1]octane derivatives of formula I are prepared by methods well known in the art of organic chemistry, see for example, J. March, 'Advanced Organic Chemistry' 4th Edition, John Wiley and Sons. During synthetic sequences it may be necessary and/or desirable to protect sensitive or reactive groups on any of the molecules concerned. This is achieved by means of conventional protecting groups, such as those described in T.W. Greene and P.G.M. Wutts 'Protective Groups in Organic Synthesis' 3rd Edition, John Wiley and Sons, 1999. The protective groups are optionally removed at a convenient subsequent stage using methods well known in the art.

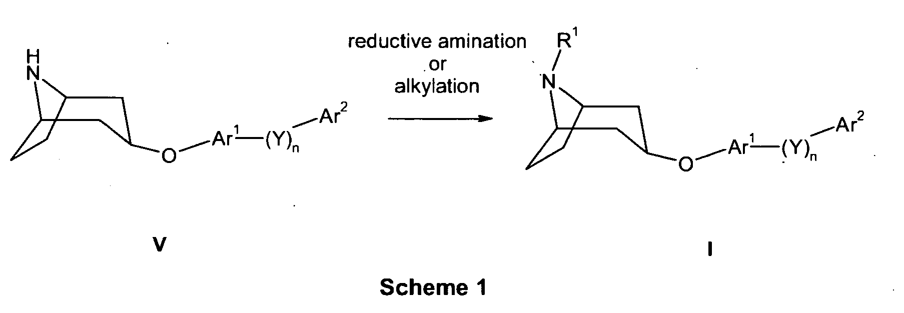

Compounds of formula **I,** wherein R¹, n, Y, Ar¹ and Ar² have the meanings as previously defined (except that R¹ is not H), are prepared from compounds of formula **V** by reductive amination with suitable aldehydes in the presence of a suitable reducing agent such as sodium cyanoborohydride or sodium triacetoxyborohydride in a suitable solvent such as methanol. Alternatively, for example, compounds of formula **I** can be prepared by alkylation of compounds of formula **V** with an alkyl halide or an alkyl sulphonate in the presence of a base such as potassium carbonate or cesium carbonate in a suitable solvent such as acetonitrile or ethyl acetate **(Scheme 1).**

Compounds of general formula **V** are prepared by substitution of the hydroxyl group in 8-azabicyclo[3.2.1]octan-3-α-ol or 8-azabicyclo[3.2.1]octan-3-β-ol, temporarily protected on nitrogen by a protecting group PG such as the acid labile t-butyloxycarbonyl (Boc) group, with compounds of formula **VI** wherein n, Y, Ar¹ and Ar² have the meanings as previously defined. For example, the substitution reaction is effected using the Mitsunobu reaction with the aid of coupling reagents known in the art of organic chemistry, such as DEAD (diethylazodicarboxylate) and PPh₃ (triphenylphosphine), ADDP (1,1'-(azodicarbonyl)dipiperidine) and PBu₃ (tributylphosphine), or (4,4-Dimethyl-1,1-dioxido-1,2,5-thiadiazolidin-2-yl)triphenylphosphonium, in solvents such as THF, or DCM to afford *N-*protected 3-substituted-8-azabicyclo[3.2.1]octanes of formula **VII.** Treatment of compounds of formula **VII** with suitable reagents to remove the protecting group, such as trifluoroacetic acid to remove the t-butyloxycarbonyl (Boc) group, gives 3-substituted-8-azabicyclo[3.2.1]octanes of formula **V (Scheme 2)**.

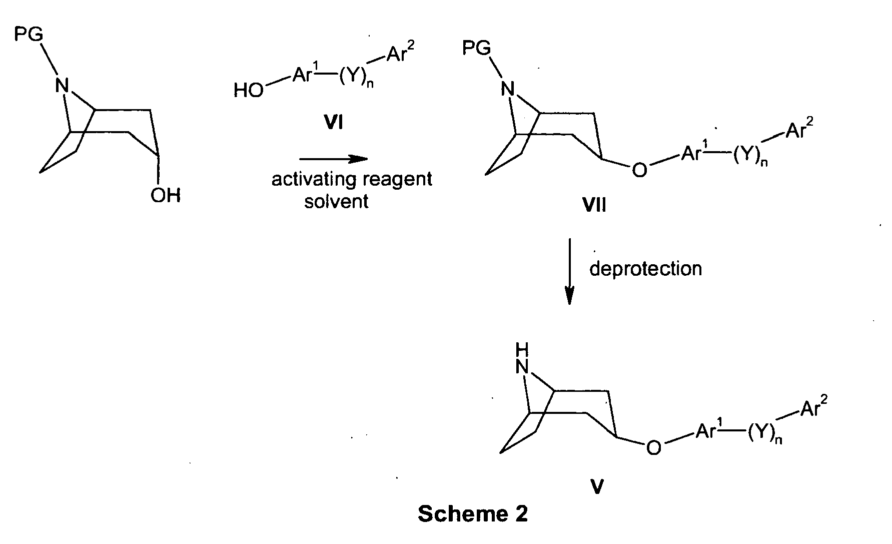

Alternatively, compounds of general formula **VII** are prepared by displacement of a leaving group from a 3-substituted-8-azabicyclo[3.2.1 ]octane **VIII**, where LG is a suitable leaving group, and PG is a suitable protecting group such as the acid labile t-butyloxycarbonyl (Boc) group, with compounds of formula **VI** using a suitable base such as sodium hydride, in a solvent such as DMF. Suitable leaving groups are, for example, a halide or an alkyl or aryl sulphonate **(Scheme 3)**.

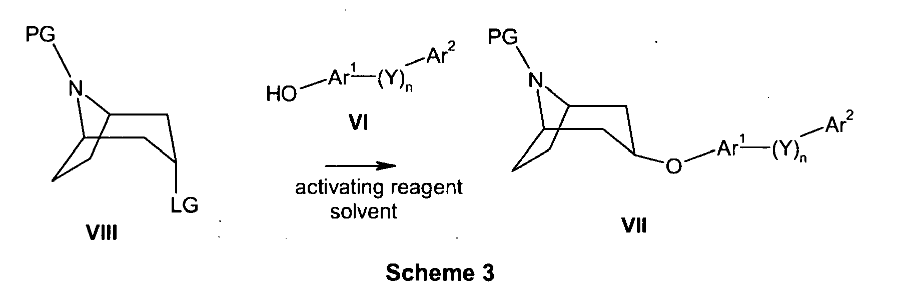

Alternatively, it will be readily appreciated by one skilled in the art that the compounds of general formula **VII** may be prepared by a nucleophilic aromatic substitution reaction involving displacement of a halogen, for example fluorine, from a compound such as **IX,** wherein n, Y, Ar¹ and Ar² have the meanings as previously defined and Hal is a halogen, with the alkoxide of 8-azabicyclo[3.2.1]octan-3-α-ol or 8-azabicyclo[3.2.1]octan-β-ol temporarily protected on nitrogen by a protecting group PG. Suitable bases for the formation of the alkoxide include, for example, sodium hydride in a solvent such as DMF **(Scheme 4).**

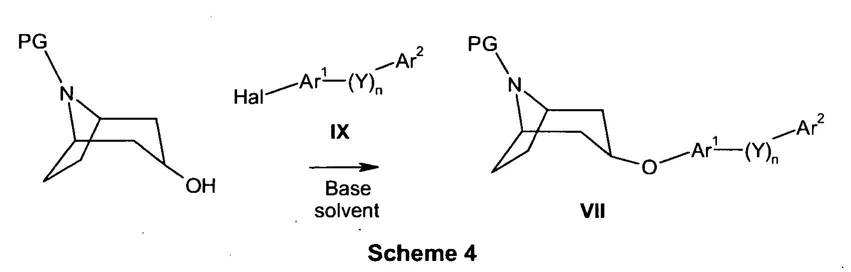

Compounds of general formula **VI** wherein n Y, Ar¹ and Ar² have the meanings as previously defined, are obtained from commercial sources, or are prepared by literature procedures or modifications of literature procedures known to persons skilled in the art. For example, in the case where n is 0, compounds of general formula **XI** are prepared by Suzuki reaction of a suitable aryl or heteroaryl boronic acid **X** with a suitable aryl or heteroaryl halide or triflate **(a) Scheme 5,** wherein Q is a halide or triflate. Furthermore, it will be readily appreciated by one skilled in the art that the compounds of general formula **XI** are also prepared using the opposite coupling partners, for example **(b) Scheme 5.** Alternatively compounds of general formula **VI** may be prepared by a variety of metal catalyzed Carbon-Carbon bond forming reactions well known to those skilled in the art - see for example Ei-ichi Negishi (Editor), Armin de Meijere (Associate Editor) Handbook of Organopalladium Chemistry for Organic Synthesis, John Wiley and Sons, 2002.

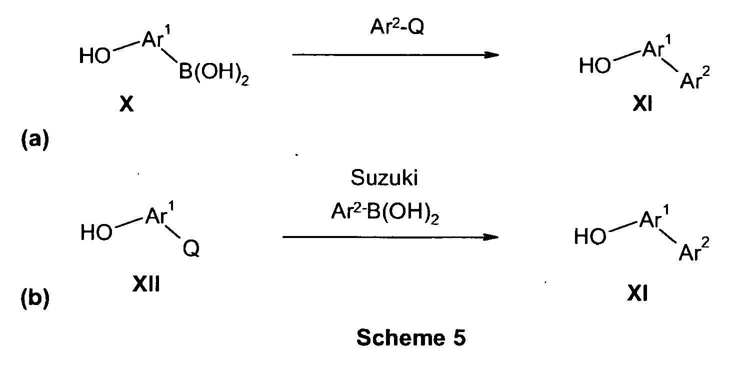

Compounds of general formula **VI,** wherein n is 1 and Y is O, are prepared from a boronic acid **XIII** wherein PG is a suitable protecting group such as a methyl ether, with a suitable nucleophile Ar²-OH, wherein Ar² has the meaning previously defined, using methods previously described in the literature (Steven V. Ley, Andrew W. Thomas, Angewandte Chemie International Edition, 2003, Volume 42, Issue 44, 5400-5449). Treatment of compounds of formula **XIV** with suitable reagents to remove the protecting group, such as pyridine hydrochloride or boron tribromide to deprotect the methyl ether, gives compounds of formula **VI,** wherein Y is O and n is 1 **(Scheme 6).** Furthermore, it will be readily appreciated by one skilled in the art that the compounds of general formula **VI** can also be prepared using the opposite coupling partners, for example **(b) Scheme 6**

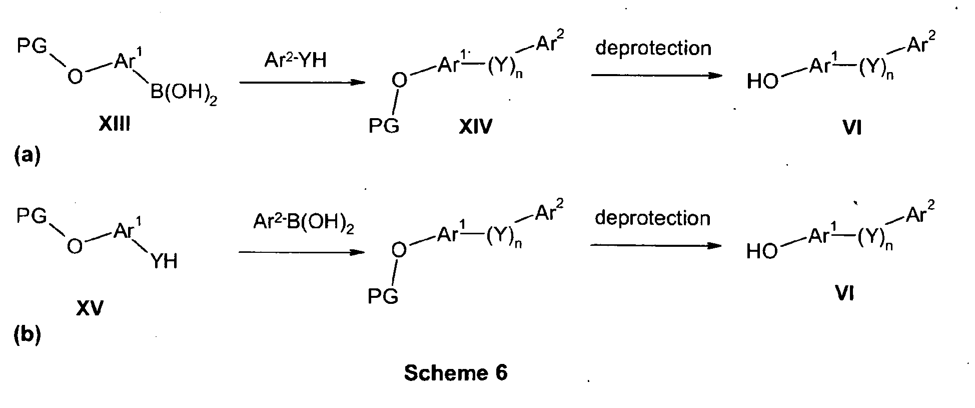

Alternatively, compounds of general formula **VI,** wherein n is 1 can be prepared by Ullmann coupling of a suitable nucleophile such as phenol and a suitable aryl or heteroaryl of formula **XVI** wherein Q is halide or triflate, temporarily protected by a protecting group PG. Suitable catalysts include CuBr with a base such as cesium carbonate in a suitable polar aprotic solvent such as DMF **(a) Scheme 7.** It will be readily appreciated by one skilled in the art that the compounds of general formula **VI** may also be prepared by a nucleophilic aromatic substitution reaction of a suitable nucleophile such as phenol with a suitable electrophile such as formula **XVI** wherein Q is a halogen, preferably fluoro, temporarily protected by a protecting group PG. Suitable bases include sodium hydride, in a polar aprotic solvent such as DMF. Furthermore, one skilled in the art will readily appreciate that compounds of the general formula **VI** are also prepared using the opposite coupling partners for example **(b) Scheme 7,** wherein n, Y, Ar¹, Ar², PG and Q have the meanings previously defined.

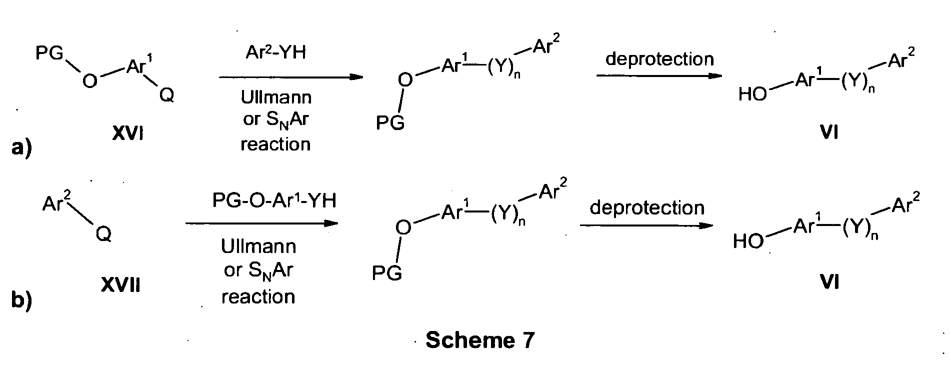

Compounds of general formula **X, XII, XIII, XV, XVI** and **XVII** are obtained from commerical sources, or are prepared by literature procedures or modifications of literature procedures known to persons skilled in the art. For example, by electrophillic aromatic substitution such as bromination with bromine or *N-*bromosuccinimide; chlorination with, for example, *N-*chlorosuccinimide and trifluoroacetic acid in a solvent such as DCM; diazotisation and then hydrolysis of commercially available aniline precursors using sodium nitrite and conc. sulphuric acid, or cyanation for example using zinc (II) cyanide with a suitable catalyst such as tetrakis(triphenylphosphine)palladium (0) in a suitable solvent such as DMF. See for example Leo Paquette, Editor-in-Chief, Encyclopedia of Reagents for Organic Synthesis, John Wiley and Sons, 1995.

It will be readily appreciated by one skilled in the art that the compounds of general formula **I** can be prepared using the general procedures and/or reaction sequences described above in any suitable order. For example, whereas the processes detailed above describe coupling of the Ar¹-Q and (HO)₂B-Ar² groups prior to Mitsunobu coupling, it will be recognized that, in some cases, the Ar¹-Q and (OH)₂B-Ar² or the Ar¹-B(OH)₂ and Ar²-Q groups, can be coupled after the substitution reaction with 8-azabicyclo[3.2.1]octan-3-α-ol.

The present invention also includes within its scope all stereoisomeric forms of a 8-azabicyclo[3.2.1]octane derivative as disclosed herein. In particular, the invention includes both *exo* and *endo* stereoisomers resulting when the 3-substituent is in the *exo* and *endo* configuration respectively. In the case of individual stereoisomers of compounds of formula I or salts or solvates thereof, the present invention includes the aforementioned stereoisomer substantially free, *i.e.*, associated with less than 5%, preferably less than 2% and in particular less than 1% of the other stereoisomer. Mixtures of stereoisomers in any proportions are also included within the scope of the present invention.

The present invention also includes within its scope all isotopically labelled forms of the 8-azabicyclo[3.2.1]octane derivatives disclosed herein. For example, compounds isotopically labelled with ²H, ³H, ¹¹C, ¹³C, ¹⁴C, ¹³¹I, ¹²⁵I, ¹²³I and ¹⁸F. The labelled compounds are useful as diagnostic tools, radio tracers, or monitoring agents in various diagnostic methods and for *in vivo* receptor imaging.

The present invention also includes within its scope the 8-azabicyclo[3.2.1]octane derivatives of the present invention in the form as a free base and in the form of a pharmaceutically acceptable salt. These salts are also obtained by treatment of said free base with an organic or inorganic acid such as hydrogen chloride, hydrogen bromide, hydrogen iodide, sulfuric acid, phosphoric acid, acetic acid, trifluoroacetic acid, propionic acid, glycolic acid, maleic acid, malonic acid, methanesulphonic acid, fumaric acid, succinic acid, tartaric acid, citric acid, benzoic acid and ascorbic acid. All salts, whether pharmaceutically acceptable or not are included within the scope of the present invention.

The 8-azabicyclo[3.2.1]octane derivatives of the present invention exist in both solvated and unsolvated forms, including hydrated forms. Both these forms are encompassed within the scope of the present invention.

The 8-azabicyclo[3.2.1]octane derivatives of the present invention also exist as amorphous forms. Multiple crystalline forms are also possible. All these physical forms are included within the scope of the present invention.

The 8-azabicyclo[3.2.1]octane derivatives of the present invention are, *inter alia,* neurotransmitter reuptake inhibitors as demonstrated *in vitro* by their ability to inhibit the reuptake of one or more of serotonin, noradrenaline and dopamine in cells stably transfected with the human serotonin, noradrenaline and dopamine transporters. Consequently, the 8-azabicyclo[3.2.1]octane derivatives of the present invention are useful in therapy. As such, the 8-azabicyclo[3.2.1]octane derivatives of the present invention are useful in the manufacture of a medicament for the treatment or prevention of diseases for which the reuptake inhibition of one or more monoamines contributes to the therapeutic effect. In a further embodiment, the 8-azabicyclo[3.2.1]octane derivatives of the present invention are useful for the manufacture of a medicament for the treatment or prevention of a disease or disorder of the nervous system, both centrally and peripherally which is responsive to monoamine neurotransmission reuptake.

In a further aspect the 8-azabicyclo[3.2.1]octane derivatives of the present invention are useful for the treatment or prevention of depression, anxiety, pain, panic disorders, attention deficit hyperactivity disorder (ADHD), or obsessive compulsive disorder. Depression states in the treatment of which the 8-azabicyclo[3.2.1]octane derivatives of the present invention and their pharmaceutically acceptable salts and solvates are particularly useful are those classified as mood disorders in the Diagnostic and Statistical Manual of Mental Disorders, Fourth Edition- Text Revised, American Psychiatric Association, Washington D.C. (2000), including mood episodes, depressive disorders, bipolar disorders and other mood disorders.

The present application discloses a method for the treatment of a mammal, including a human, suffering from or liable to suffer from any of the aforementioned diseases or disorders, which method comprises administering an effective amount of a 8-azabicyclo[3.2.1]octane derivative of the present invention or a pharmaceutically acceptable salt or solvate thereof.

The amount of a 8-azabicyclo[3.2.1]octane derivative of the present invention or a pharmaceutically acceptable salt or solvate thereof, also referred to herein as the active ingredient, which is required to achieve a therapeutic effect will, of course, vary with the particular compound, the route of administration, the age and condition of the recipient, and the particular disorder or disease being treated.

A suitable daily dose for any of the above mentioned disorders will be in the range of 0.001 to 50 mg per kilogram body weight of the recipient (*e.g*. a human) per day, preferably in the range of 0.01 to 20 mg per kilogram body weight per day. The desired dose may be presented as multiple sub-doses administered at appropriate intervals throughout the day.

Whilst it is possible for the active ingredient to be administered alone, it is preferable to present it as a pharmaceutical formulation. The present invention therefore also provides a pharmaceutical composition comprising a 8-azabicyclo[3.2.1]octane derivative according to the present invention in admixture with one or more pharmaceutically acceptable excipients, such as the ones described in Gennaro et al., Remmington: The Science and Practice of Pharmacy, 20th Edition, Lippincott, Williams and Wilkins, 2000; see especially part 5: pharmaceutical manufacturing. Suitable excipients are described *e.g*., in the Handbook of Pharmaceutical Excipients, 2nd Edition; Editors A. Wade and P.J.Weller, American Pharmaceutical Association, Washington, The Pharmaceutical Press, London, 1994. Compositions include those suitable for oral, nasal, topical (including buccal, sublingual and transdermal), parenteral (including subcutaneous, intravenous and intramuscular) or rectal administration.

The mixtures of a 8-azabicyclo[3.2.1]octane derivative according to the present invention and one or more pharmaceutically acceptable excipient or excipients may be compressed into solid dosage units, such as tablets, or be processed into capsules or suppositories. By means of pharmaceutically suitable liquids the compounds can also be applied as an injection preparation in the form of a solution, suspension, emulsion, or as a spray, *e.g*., a nasal or buccal spray. For making dosage units *e.g*., tablets, the use of conventional additives such as fillers, colorants, polymeric binders and the like is contemplated. In general, any pharmaceutically acceptable additive can be used. The 8-azabicyclo[3.2.1]octane derivatives of the present invention are also suitable for use in an implant, a patch, a gel or any other preparation for immediate and/or sustained release.

Suitable fillers with which the pharmaceutical compositions can be prepared and administered include lactose, starch, cellulose and derivatives thereof, and the like, or mixtures thereof used in suitable amounts.

The following Examples further illustrate the compounds of the present invention and methods for their synthesis. The following examples are put forth so as to provide those of ordinary skill in the art with disclosure and description of how compounds, compositions and methods herein are made and evaluated, and are intended to be purely exemplary of the invention and are not intended to limit the scope of what the inventors-regard as their invention. Unless indicated otherwise, percent is percent by weight given the component and the total weight of the composition, temperature is in °C or is at ambient temperature, and pressure is at or near atmospheric. Commercial reagents were used without further purification.

### Methods

General Chemical Procedures. All reagents were either purchased from common commercial sources or synthesised according to literature procedures using commercial sources. Mass spectra were recorded on a Shimadzu LC-8A (HPLC) PE Sciex API 150EX LCMS. Analytical reversed-phase LCMS analysis was carried out on LUNA C18 column (5µ; 30 x 4.6 mm) under gradient conditions (90% water / 0.1% formic acid to 90% acetonitrile / 0.1 % formic acid) at a flow rate of 4 mL/min. SCX (strong cation exchange) cartridges were purchased from Phenomenex.

### Abbreviations

Dimethylformamide (DMF), dichloromethane (DCM), dimethylsulfoxide (DMSO), tetrahydrofuran (THF), high pressure liquid chromatography (HPLC), diisopropylethylamine (DIPEA), triethylamine (TEA), trifluoroacetic acid (TFA), tertbutyloxycarbonyl (Boc), ethylene glycol dimethyl ether (DME), dimethylacetamide (DMA) preparative LCMS refers to preparative high pressure liquid chromatography with mass spectrometric detection.

In the following section, examples of the synthesis of precursors and common intermediates for compounds of the present invention are described.

### (4,4-Dimethyl-1,1-dioxido-1,2,5-thiadiazolidin-2-yl)triphenylphosphonium

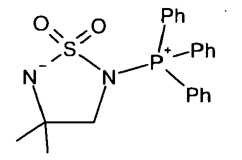

(4,4-Dimethyl-1,1-dioxido-1,2,5-thiadiazolidin-2-yl)triphenylphosphonium was prepared as described in J. Org. Chem., 1994, 59, 2289-2291

### 3-endo-Hydroxy-8-azabicyclo[3.2.1]octane-8-carboxylic acid tert-butyl ester

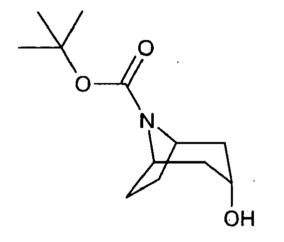

*endo*-8-Azabicyclo[3.2.1]octan-3-ol (3.6g, 28.3mmol) was dissolved in DCM (50ml) and cooled to 0°C. Triethylamine (7.8ml, 56.7mmol) was added followed by addition of di-*tert-*butyl dicarbonate (7.4g, 33.9mmol). The reaction mixture was warmed to ambient temperature and stirred for 12h. The reaction mixture was diluted with water. The organic layer was separated and washed with saturated citric acid (aq), water and brine. The organic layer was dried over MgSO₄ and concentrated *in vacuo* to afford 3-*endo*-hydroxy-8-azabicyclo[3.2.1]octane-8-carboxylic acid *tert*-butyl ester as a white solid (6.45g, 28.4mmol, 100%). M.S. (ESI) (m/z): 228 [M+H]⁺

### 3-exo-Hydroxy-8-azabicyclo[3.2.1]octane-8-carboxylic acid tert-butyl ester

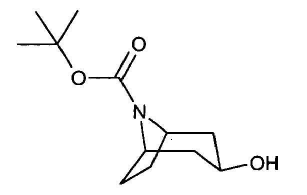

Diethylazodicarboxylate (1.74mL, 11mmol) was added dropwise to a solution of 3-*endo-*hydroxy-8-azabicyclo[3.2.1]octane-8-carboxylic acid *tert*-butyl ester (2.09g, 9.2mmol), triphenylphosphine (2.89g, 11mmol) and 4-nitrobenzoic acid (1.69g, 10mmol) in THF (25mL). The reaction mixture was stirred under a nitrogen atmosphere for 18h at ambient temperature. Volatiles were removed under reduced pressure and the residue was purified by chromatography on silica gel. Elution with DCM afforded the ester as a pale yellow solid (2.6g, 6.9mmol, 75%). 4N sodium hydroxide (aq) (3.6mL, 14mmol) was added to a solution of the ester in THF (25mL) and the reaction mixture stirred at ambient temperature for 3 days. Diethyl ether (35mL) and water (10mL) were added to the reaction mixture. 2N sodium hydroxide (aq) solution was added to the organic layer which was removed, dried over. Na₂SO₄ and the solvent evaporated *in vacuo*. The crude material was purified by chromatography on silica gel. Elution with DCM with a gradient to 5:95 methanol:DCM afforded 3-*exo*-hydroxy-8-azabicyclo[3.2.1]octane-8-carboxylic acid *tert-*butyl ester (1.4g, 6.2mmol, 89%) M.S. (ESI) (m/z): 228[M+H]⁺

### 3-exo-Methanesulfonyloxy-8-azabicyclo[3.2.1]octane-8-carboxylic acid tert-butyl ester

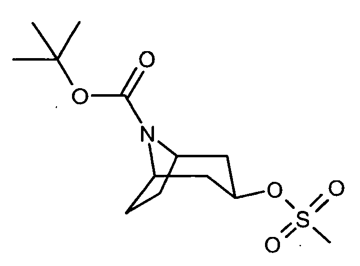

A solution of 3-*exo*-hydroxy-8-azabicyclo[3.2.1]octane-8-carboxylic acid *tert*-butyl ester (230mg, 1.0mmol) and triethylamine (0.148mL, 1.1mmol) in DCM (4mL) was stirred under a nitrogen atmosphere and cooled to 0°C. Methanesulfonyl chloride (0.082mL, 1.1 mmol) was added dropwise to the reaction mixture, which was allowed to rise to ambient temperature. The reaction mixture was stirred for a further 18h. The reaction mixture was evaporated *in vacuo* and the residue purified by chromatography on silica gel. Elution with DCM with a gradient to 2:98 acetone:DCM afforded 3-*exo*-methanesulfonyloxy-8-azabicyclo[3.2.1]octane-8-carboxylic acid *tert*-butyl ester (298mg, 0.98mmol, 97%).

### 3-endo-Methanesulfonyloxy-8-azabicyclo[3.2.1]octane-8-carboxylic acid tert-butyl ester

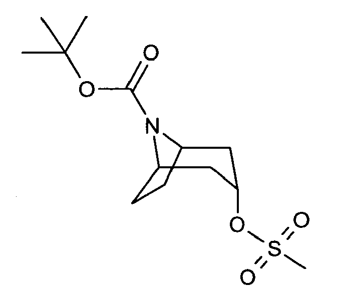

A solution of 3-*endo*-hydroxy-8-azabicyclo[3.2.1]octane-8-carboxylic acid *tert*-butyl ester (5.0g, 22mmol) and triethylamine (3.3mL, 24mmol) in dry DCM (40mL) was stirred under an argon atmosphere and cooled to 0°C. Methane sulfonyl chloride (1.85mL, 24mmol) was added dropwise to the reaction mixture, which was allowed to warm to ambient temperature. The reaction mixture was stirred for a further 18h, and the reaction mixture quenched by the addition of water. The organic layer was separated, dried over Na₂SO₄, absorbed onto silica gel and purified by chromatography on silica gel. Elution with 1:4 ethyl acetate:heptane afforded 3-*endo*-methanesulfonyloxy-8-azabicyclo[3.2.1]octane-8-carboxylic acid *tert*-butyl ester (4.0g, 13mmol, 60%).

### 4-Bromo-3-phenoxyphenol and 2,4-Dibromo-5-phenoxyphenol

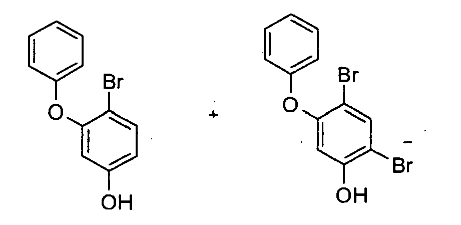

3-Phenoxyphenol (700mg, 3.8mmol) was stirred at 0°C in DCM (7mL) and bromine (0.154mL, 3.0mmol) was added dropwise. The reaction was stirred at ambient temperature for 10min and then the solvents removed *in vacuo* to afford a crude oil which was purified by preparative HPLC to afford, in order of elution, 4-bromo-3-phenoxyphenol (126mg, 0.5mmol, 16%) M.S. (ESI) (m/z): 263,265 [M-H]⁻; and 4,6-dibromo-3-phenoxyphenol (201mg, 0.6mmol, 39%) M.S. (ESI) (m/z): 341,343,345 [M-H]⁻

Similarly prepared were:
**6-Bromobiphenyl-3-ol**
**4-Bromobiphenyl-3-ol**

### 4-Chloro-3-phenoxyphenol and 2-Chloro-5-phenoxyphenol

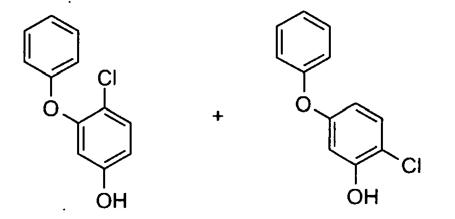

3-Phenoxyphenol (1.00g, 5.4mmol) was dissolved in MeCN (40mL) and TFA (0.5mL). *N*-Chlorosuccinimide (717mg, 5.4mmol) was added and the reaction stirred at ambient temperature for 48h. Solvents were removed *in vacuo* to afford the crude product as an oil (2.15g). 300mg of this crude material was purified by HPLC to afford in order of elution 4-chloro-3-phenoxyphenol (68mg, 0.31 mmol, 41%) M.S. (ESI) (m/z): 219,221 [M-H]⁻; and 6-chloro-3-phenoxyphenol (73mg, 0.33mmol, 44%) M.S. (ESI) (m/z): 219,221 [M-H]⁻.

Similarly prepared were:.
**6-Chlorobiphenyl-3-ol**
**4-Chlorobiphenyl-3-ol**

### 4-Chloro-3-(4-fluorophenoxy)phenol

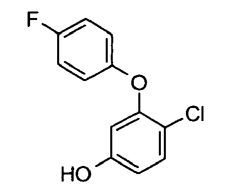

2-Bromo-1-chloro-4-methoxybenzene (200mg, 0.9mmol), cesium carbonate (588mg, 1.8mmol), 4-fluorophenol (202mg, 1.8mmol), Cul (17mg, 0.09mmol) and *N-*methylpyrrolidine (1 mL) were sealed in a microwave vessel and heated in a microwave at 200°C for 1800s. The crude reaction mixture was purified by chromatography on silica gel. Elution with 10:90 ethyl acetate:heptane with a gradient to 30:70 ethyl acetate:heptane afforded 1-chloro-2-(4-fluorophenoxy)-4-methoxybenzene (112mg, 0.44mmol, 49%). 1-Chloro-2-(4-fluorophenoxy)-4-methoxybenzene (110mg, 0.44mmol) prepared above, was dissolved in DCM (5.00mL) and stirred under argon at -78°C. Boron tribromide (1.0M in DCM, 2.20mL) was then added and the reaction stirred at -78°C for 1 h. The reaction was allowed to warm to ambient temperature overnight, quenched with saturated Na₂CO₃ (aq) and then the aqueous and organic layers were separated. The solvent was removed *in vacuo* and the crude product was purified by chromatography on silica gel. Elution with 2:98 ethyl acetate:heptane with a gradient to 20:80 ethyl acetate:heptane afforded 4-chloro-3-(4-fluorophenoxy)phenol (92mg, 0.38mmol, 87%). M.S. (ESI) (m/z): 237, 239 [M-H]⁻

### 5-Bromo-2-methylphenol

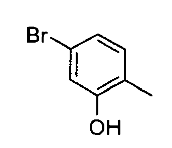

A solution of concentrated sulphuric acid (6mL) in distilled water (75mL) was added to 5-bromo-2-methylaniline (1 g, 5.38mmol). The resultant suspension was heated to 90°C and stirred for 4.5h. The reaction mixture was then cooled using an ice bath and a solution of sodium nitrite (384mg, 5.57mmol) in water (5mL) was added to the reaction mixture at 0°C. The reaction was then allowed to warm to ambient temperature. The reaction mixture was then added to a solution of concentrated sulphuric acid (6mL) in water (75mL) which had been preheated to 90°C. The reaction mixture was stirred for 1 h at 90°C and allowed to cool, on standing, overnight. A precipitate was observed in the reaction mixture. The precipitate was collected by filtration, washed with water, and dried in a vacuum oven to afford .5-bromo-2-methylphenol as a brown solid (510mg, 2.73mmol, 51%). M.S. (ESI) (m/z) 185, 187[M-H]⁻

Similarly prepared were
**3**-**Fluoro-5-iodophenol**

### 5-Fluorobiphenyl-3-ol

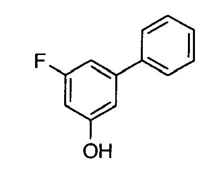

Palladium (II) acetate (2.4mg, 0.01 mmol) was added to a suspension of 3-fluoro-5-iodophenol (250mg, 1.05mmol), phenylboronic acid (154mg, 1.26mmol) and sodium carbonate (3.34g, 3.15mmol) in water (5mL), under an atmosphere of nitrogen. The reaction mixture was stirred at ambient temperature for 16h. The reaction mixture was then filtered through a plug of dicalite to remove black precipitate. The dicalite was washed with MeOH. The filtrate was diluted with water (250mL) and then adjusted to pH2 using 2M HCl (aq). The product was extracted into DCM, dried over Na₂SO₄ and concentrated *in vacuo.* The crude material was purified by chromatography on silica gel. Elution with 10:90 ethyl acetate:heptane afforded 5-fluorobiphenyl-3-ol (186mg, 0.988mmol, 94%) M.S. (ESI) (m/z): 187 [M-H]⁻.

Similarly prepared were:.
**6-Fluorobiphenyl-3-ol**
**5-Bromobiphenyl-3-ol**
**[1,1';3',1"]Terphenyl-5'-ol**
**5.Chlorobiphenyl-3-ol**
**3-Thiophen-2-ylphenol**

### 4-Phenylpyridin-2-ol

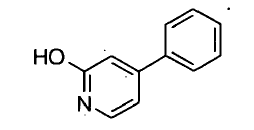

Tetrakis(triphenylphosphine)palladium (0) (164mg, 0.14mmol) was added to a stirred suspension of phenylboronic acid (345mg, 2.84mmol) and 4-bromo-2-hydroxypyridine (494mg, 2.84mmol) in DME (10mL). The reaction mixture was stirred under an argon atmosphere for 90min. Degassed 2N potassium carbonate solution (aq) (10mL) was added and the reaction mixture stirred with heating at 90°C for 72h. The solvent was evaporated *in vacuo,* and the residue partitioned between ethyl acetate and water. The aqueous layer was washed with ethyl acetate (x2). The organics were combined, dried over Na₂SO₄ and the solvent evaporated *in vacuo.* The residue was purified by chromatography on silica gel. Elution with DCM with a gradient to 5:95 methanol:DCM afforded 4-phenylpyridin-2-ol (311mg, 1.82mmol, 64%) M.S. (ESI) (m/z): 172 [M+H]⁺.

Similarly prepared were:.
**2-Phenylpyridin-4-ol**
**6-Phenylpyridin-2-ol**
**2'-Chlorobiphenyl-3-ol**
**3'-Chlorobiphenyl-3-ol**
**4'-Chlorobiphenyl-3-ol**
**4'-Trifluoromethylbiphenyl-3-ol**
**3'-Trifluoromethoxybiphenyl-3-ol**
**4'-Trifluoromethoxybiphenyl-3-ol**
**2'-Methylbiphenyl-3-ol**
**3'-Methylbiphenyl-3-ol**
**3'-Hydroxybiphenyl-2-carbonitrile**
**3'-Hydroxybiphenyl-4-carbonitrile**
**2'-Fluorobiphenyl-3-ol**
**3'-Fluorobiphenyl-3-ol**
**2'-Trifluoromethylbiphenyl-3-ol**
**3'-Hydroxybiphenyl-3-carbonitrile**
**3-Pyridin-3-ylphenol**
**4-Chlorobiphenyl-3-ol**
**3'-Methoxybiphenyl-3-ol**
**2'-Fluorobiphenyl-3-ol**
**3'-Trifluoromethylbiphenyl-3-ol**
**4'-Methoxybiphenyl-3-ol**
**2'-Trifluoromethylbiphenyl-3-ol**
**2'-Methoxybiphenyl-3-ol**
**3'-Methoxybiphenyl-3-ol**
**3'-Hydroxybiphenyl-3-carbonitrile**

### 5-Hydroxybiphenyl-3-carbonitrile

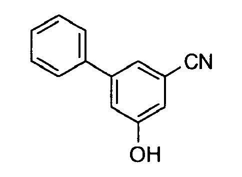

Tetrakis(triphenylphosphine)palladium (0) (116mg, 0.1mmol) was added to 5-bromobiphenyl-3-ol (250mg, 1mmol) and zinc cyanide (117mg, 1.0mmol) dissolved in DMF (5mL) in a microwave vessel. The reaction vessel was sealed and then heated at 200°C for 300 seconds. The reaction was quenched with water and the product was extracted into DCM. The solution was filtered and then concentrated *in vacuo*. The crude material was then purified by chromatography on silica gel. Elution with 10:90 ethyl acetate:heptane afforded 5-hydroxybiphenyl-3-carbonitrile (74mg, 0.379mmol, 38%). M.S. (ESI) (m/z): 194 [M-H]⁻.

Similarly prepared were:
**5-Hydroxybiphenyl-2-carbonitrile**

### 2-Methyl-3-phenoxyphenol

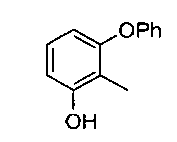

1-Bromo-3-fluoro-2-methylbenzene (388mg, 2.05mmol), and (4-methoxyphenyl)methanol (0.511mL, 4mmol) were dissolved in DMF (5mL) in a microwave vessel. Sodium hydride as a 60% dispersion in mineral oil (164mg, 4.0mmol) was added portionwise under a flow of nitrogen over 10min. The reaction vessel was then sealed and heated at 180°C for 900s. The crude reaction mixture was poured onto 1:1 water:brine (6mL) and DCM (10mL). The biphasic mixture was stirred at ambient temperature for 1h and then the organic and aqueous layers were separated. The combined organics were dried over Na₂SO₄ and concentrated *in vacuo.* The crude material was then purified by chromatography on silica gel. Elution with 5:95 ethyl acetate:heptane afforded 1-bromo-3-(4-methoxybenzyloxy)-2-methylbenzene (289mg, 0.9mmol, 46%).

Sodium hydride as a 60% dispersion in mineral oil (56mg, 1.4mmol) was added portionwise to a solution of phenol (0.35M in DMF, 2mL). The resultant solution was added to a mixture of 1-bromo-3-(4-meth oxybenzyloxy)-2-methyl benzene (215mg, 0.7mmol) and cesium carbonate (228mg, 0.7mmol) in a microwave vessel. Copper (I) bromide (10mg) was added, the vessel was sealed and the reaction heated at 180°C for 900s. 1 N NaOH (aq) (2mL) and DCM (5mL) were added to the cooled reaction mixture and the organic layer was separated and concentrated *in vacuo.* The crude material was then purified by chromatography on silica gel. Elution with 5:95 ethyl acetate:heptane afforded 1-(4-methoxybenzyloxy)-2-methyl-3-phenoxybenzene (205mg, 0.64mmol, 91%).

1-(4-Methoxybenzyloxy)-2-methyl-3-phenoxybenzene (205mg, 0.64mmol) was dissolved in ethanol (5mL) and palladium on charcoal 10%wt (60mg) was added. The reaction mixture was agitated under an atmosphere of hydrogen (1atm) for 72h. The crude reaction mixture was filtered over dicalite and the solvents removed *in vacuo*. The crude product was taken up in DCM (4mL) and 1 N NaOH (aq) (4mL) and the aqueous layer was separated, then acidified to pH 1 with 5N HCl (aq). The aqueous layer was extracted with DCM (10mL) and the combined organics were concentrated *in vacuo*. The crude material was then purified by chromatography on silica gel. Elution with 5:95 ethyl acetate:heptane afforded 2-methyl-3-phenoxyphenol (43mg, 0.21 mmol, 34%).

Similarly prepared were:.
**2,3-Dimethyl-5-phenoxyphenol**
**3-Phenoxy-4-trifluoromethylphenol**
**3-Phenoxy-5-trifluoromethylphenol**
**4-Methyl-3-phenoxyphenol**
**2-Fluoro-3-methoxy-5-phenoxyphenol**

### 5-Phenylpyridin-3-ol

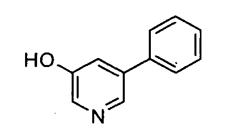

Tetrakis(triphenylphosphine)palladium(0) (242mg, 0.21 mmol) was added to a stirred solution of phenyl boronic acid (510mg, 4.18mmol) and 3-bromo-5-methoxypyridine (786mg, 4.18mmol) in DME (10mL). The reaction mixture was stirred under an argon atmosphere for 30min. Degassed potassium carbonate (aq) (10mL) was added and the reaction mixture stirred with heating at 90°C for 18h. The solvent was evaporated *in vacuo,* and the residue partitioned between ethyl acetate and water. The aqueous layer was washed with ethyl acetate. The organics were combined, dried over Na₂SO₄ and the solvent evaporated *in vacuo.* The residue was purified by chromatography on silica gel. Elution with 10:90 ethyl acetate:isohexane with a gradient to 15:85 ethyl acetate:isohexane afforded a yellow oil. The oil was dissolved in DCM, and cooled to - 78°C under a nitrogen atmosphere. Boron tribromide (1.0M in DCM, 9.15mL) was added dropwise over 20min and the reaction mixture was warmed to ambient temperature and stirred at this temperature for 18h. The resultant mixture was basified to pH8 by the dropwise addition of saturated sodium hydrogen carbonate (aq). The organics were removed and the aqueous residue extracted into ethyl acetate (x3). The organic layers were combined, dried over Na₂SO₄ and evaporated in vacuo to give 5-phenylpyridin-3-ol (245mg, 1.43mmol, 47%) M.S. (ESI) (m/z): 172 [M+H]⁺.

Similarly prepared were:
**2-Fluorobiphenyl-3-ol**
**2'-Fluoro-3'-hydroxybiphenyl-4-carbonitrile**

### 2,4-Dichloro-6-hydroxypyridine

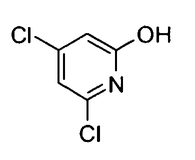

Sodium nitrite (64mg, 0.93mmol) dissolved in water (0.6 mL), was added dropwise to a stirred solution of 2-amino-4,6-dichloropyridine (prepared according to the method described in Recl. Trav. Chim. Pays-Bas, 1950, 69, 673) (126 mg, 0.77 mmol) in 5% sulphuric acid (aq) (5 mL) at 0 °C, over 5 min. The mixture was stirred at 0°C for 1 h and then diluted with water (20 mL) and extracted with DCM (3 x 20 mL). The combined extracts were washed with brine (20 mL), dried over MgSO₄ and concentrated *in vacuo* to afford the 2,4-dichloro-6-hydroxypyridine (116 mg, 0.71mmol, 92 %) M.S. (ESI) *(m*/*z)* 164,166 [M+H]⁺.

The present invention is further illustrated by the following examples:

### Procedure I

### EXAMPLE 1.1: 3-exo-(3-Phenoxyphenoxy)-8-azabicyclo[3.2.1]octane

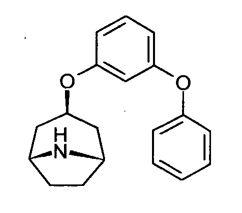

Diethylazodicarboxylate (1.89mL, 12mmol) was added dropwise to a solution of 3-*endo-*hydroxy-8-azabicyclo[3.2.1]octane-8-carboxylic acid *tert*-butyl ester (2.27g, 10mmol), triphenylphosphine (3.15g, 12mmol) and 3-phenoxyphenol (1.93mL, 12mmol) in THF (60mL). The reaction mixture was stirred under a nitrogen atmosphere for 72h at ambient temperature. Volatiles were removed under reduced pressure. The resultant material was dissolved in DCM (50mL) and TFA (5mL) added. The reaction mixture was stirred at ambient temperature for 12h. Volatiles were removed under reduced pressure, the crude product dissolved in methanol (40mL) and the solution loaded onto a SCX cartridge (20g). The cartridge was eluted with methanol (40mL) to remove triphenylphosphine oxide. Elution with ammonia in methanol (2M, 40mL) followed by evaporation *in vacuo* afforded crude 3-(3-phenoxyphenoxy)-8-azabicyclo[3.2.1]octane as an oil. The crude material was then purified by chromatography on silica gel. Elution with 98:2 DCM:MeOH with a gradient to 90:10:0.5 DCM:MeOH:ammonia, afforded the product as an oil. The product was dissolved in methanol (5mL) and treated with hydrochloric acid in methanol until the solution was acidic. The solution was concentrated *in vacuo* and diethyl ether was added to precipitate the product. The precipitate was collected by filtration and recrystallised from methanol/diethyl ether to afford 3-*exo*-(3-phenoxyphenoxy)-8-azabicyclo[3.2.1]octane as the hydrochoride salt (1.08g, 3.26mmol, 33%). M.S. (ESI) (m/z): 296 [M+H]⁺.

Similarly prepared were

### EXAMPLE 1.2: 3-exo-(4-Chloro-3-(4-fluorophenoxy)phenoxy)-8-azabicyclo[3.2.1]octane

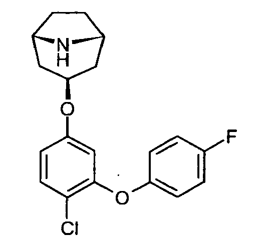

MS (ESI) (m/z): 348, 350 [M+H]⁺.

### EXAMPLE I.3: 3-exo -(5-Chlorobiphenyl-3-yloxy)-8-azabicyclo[3.2.1]octane

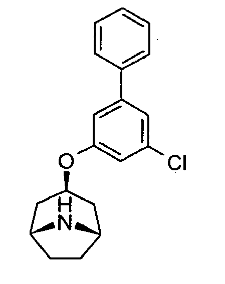

MS (ESI) (m/z): 314, 316 [M+H]⁺.

### EXAMPLE I.4: 3-exo-(5-Bromobiphenyl-3-yloxy)-8-azabicyclo[3.2.1]octane

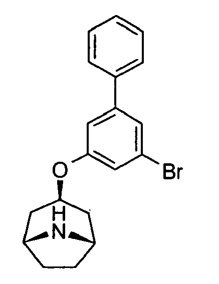

M.S. (ESI) (m/z): 358, 360 [M+H]⁺.

### EXAMPLE I.5: exo 5-(8-Azabicyclo[3.2.1]oct-3-yloxy)biphenyl-3-carbonitrile

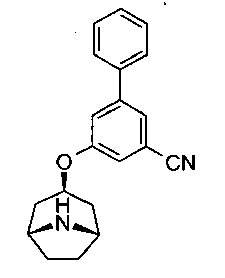

MS (ESI) (m/z): 305 [M+H]⁺.

### EXAMPLE 1.6: 3-exo-[1,1';3'1"]Terphenyl-5'-yloxy)-8-azabicyclo[3.2.1]octane

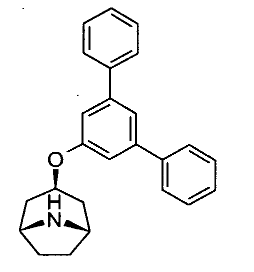

M.S. (ESI) (m/z): 356 [M+H]⁺.

### EXAMPLE 1.7: 3-exo-(5-Fluorobiphenyl-3-vloxv)-8-azabicyclo[3.2.1]octane

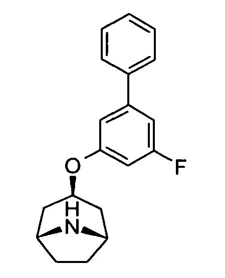

M.S. (ESI) (m/z): 298 [M+H]⁺.

### EXAMPLE 1.8: 3-exo-(6-Fluorobiphenyl-3-yloxyl-8-azabicyclo[3.2.1]octane

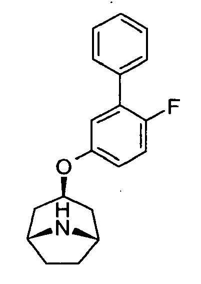

M.S. (ESI) (m/z): 298 [M+H]⁺.

### EXAMPLE 1.9: 3-exo-(Biphenyl-3-yloxy)-8-azabicyclo[3.2.1]octane

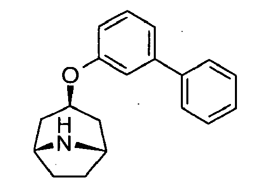

M.S. (ESI) (m/z): 280 [M+H]⁺.

### EXAMPLE I.10: 3-exo-(6-Chlorobiphenyl-3-yloxy)-8-azabicyclo[3.2.1]octane

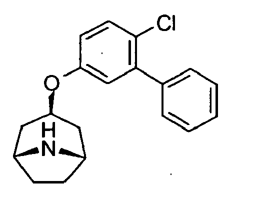

M.S. (ESI) (m/z): 314,316 [M+H]⁺.

### EXAMPLE I.11: 3-exo-{4-Chloro-3-phenoxyphenoxy)-8-azabicyclo[3.2.1]octane

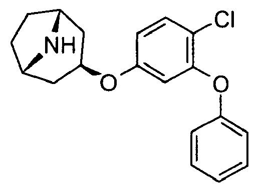

M.S. (ESI) (m/z): 330,332 [M+H]⁺.

### EXAMPLE I.12: 3-exo-(2-Chloro-5-phenoxyphenoxy)-8-azabicyclo[3.2.1]octane

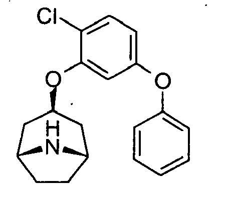

M.S. (ESI) (m/z): 330,332 [M+H]⁺.

### EXAMPLE I.13: 3-exo-(4-Bromobiphenyl-3-yloxy)-8-azabicyclo[3.2.1]octane

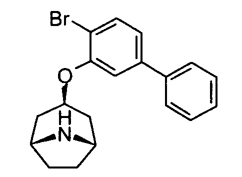

M.S. (ESI) (m/z): 358,360 [M+H]⁺.

### EXAMPLE I.14: 3-exo-(6-Bromobiphenyl-3-yloxy)-8-aza-bicyclo[3.2.1]octane

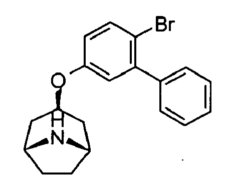

M.S. (ESI) (m/z): 358,360 [M+H]⁺.

### EXAMPLE I.15: 3-exo-(4-Bromo-3-phenoxyphenoxy)-8-azabicyclo[3.2.1]octane

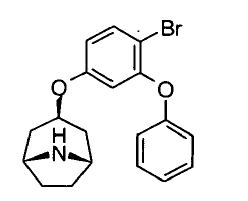

M.S. (ESI) (m/z): 374,376 [M+H]⁺.

### EXAMPLE I.16: exo 5-(8-Azabicyclo[3.2.1]oct-3 -yloxy)biphenyl-2-carbonitrile

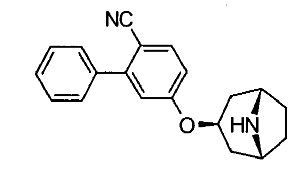

M.S. (ESI) (m/z): 305 [M+H]⁺.

### EXAMPLE 1.17: 3-exo-(Dibenzofuran-2-yloxy)-8-azabicyclo[3.2.1]octane

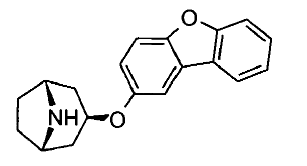

M.S. (ESI) (m/z): 294 [M+H]⁺.

### EXAMPLE 1.18: 3-exo-(3-Phenethyloxyphenoxv)-8-azabicyclo[3.2.1]octane

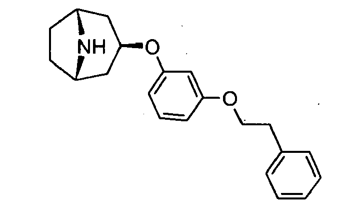

M.S. (ESI) (m/z): 324 [M+H]⁺.

### EXAMPLE I.19: 3-exo-(3-Thiophen-2-ylphenoxy)-8-azabicyclo[3.2.1]octane

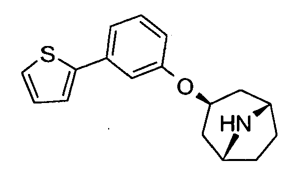

M.S. (ESI) (m/z): 286 [M+H]⁺.

### Procedure II

### EXAMPLE II.1: 3-exo-(4-Fluoro-3-phenoxyphenoxy)-8-azabicyclo[3.2.1]octane

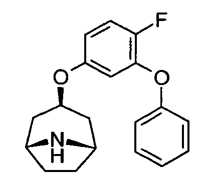

Diethylazodicarboxylate (1.65mL, 10.5mmol) was added dropwise to a solution of 3-*endo*-hydroxy-8-azabicyclo[3.2.1]octane-8-carboxylic acid *tert*-butyl ester (2.16g, 9.5mmol), triphenylphosphine (2.75g, 10.5mmol) and 4-fluoro-3-bromophenol (2.0g, 10.5mmol) in THF (95mL). The reaction mixture was stirred under a nitrogen atmosphere for 72h at ambient temperature. Volatiles were removed under reduced pressure, the residue was then triturated with 5:1 heptane:diethyl ether followed by filtration to remove precipitated triphenylphosphine oxide. The filtrate was concentrated *in vacuo*. The crude material was purified by chromatography on silica gel. Elution with 15:85 ethyl acetate:heptane, followed by washing with 1N NaOH (aq) (50mL) then concentration *in vacuo* afforded 3-*exo*-(3-bromo-4-fluorophenoxy)-8-azabicyclo[3.2.1]octane-8-carboxylic acid *tert*-butyl ester (2.18g, 5.5mmol, 57%).

3-*exo*-(3-Bromo-4-fluorophenoxy)-8-azabicyclo[3.2.1]octane-8-carboxylic acid *tert*-butyl ester (150mg, 0.37mmol), cesium carbonate (244mg, 0.75mmol), phenol (70.5mg, 0.75mmol) and copper (I) iodide (7mg, 0.04mmol) were combined with *N-*methylpyrrolidine (1mL) in a microwave vessel. The reaction was heated at 200°C for 1800s. TFA (1mL) was then added and the reaction stirred at ambient temperature for 16h. DCM (2mL) was added followed by 1 N NaOH (aq) to pH 10. The aqueous and organic layers were separated and the combined organics were concentrated *in vacuo.* The crude product was purified by preparative LCMS to afford 3-*exo*-(4-fluoro-3-phenoxyphenoxy)-8-azabicyclo[3.2.1]octane as the trifluoroacetic acid salt (20.6mg, 0.05mmol, 13%) M.S. (ESI) (m/z): 314 [M+H]⁺.

Similarly prepared were:

### EXAMPLE II.2: 3-exo-(3-(3-Chlorophenoxy)-4-fluorophenoxy)-8-azabicyclo[3.2.1]octane

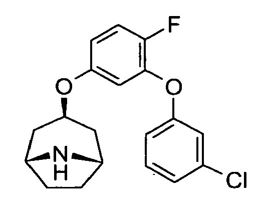

M.S. (ESI) (m/z): 348, 350 [M+H]⁺.

### EXAMPLE II.3: 3-exo-(3-(3,4-Dichlorophenoxy)-4-fluorophenoxy)-8-azabicyclo[3.2.1]octane

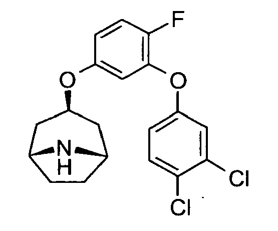

M.S. (ESI) (m/z): 382, 384 [M+H]⁺.

### EXAMPLE II.4: 3-exo-(3-(4-Chlorophenoxy)-4-fluorophenoxy)-8-azabicyclo[3.2.1]octane

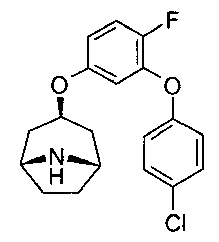

M.S. (ESI) (m/z): 348, 350 [M+H]⁺.

### EXAMPLE II.5: 3-exo-(4-Fluoro-3-(3-methoxyphenoxy)phenoxy)-8-azabicyclo[3.2.1]octane

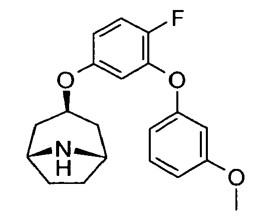

M.S. (ESI) (m/z): 344 [M+H]⁺.

### EXAMPLE II.6: exo 3-[5-(8-Azabicyclo[3.2.1]oct-3-yloxyl)-2-fluorophenoxylbenzonitrile

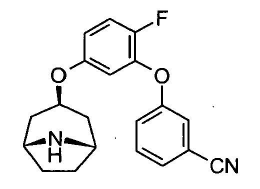

M.S. (ESI) (m/z): 339 [M+H]⁺.

### EXAMPLE II.7: exo 3-[3-(Pyridin-3-yloxy)phenoxy]-8-azabicyclo[3.2.1]octane

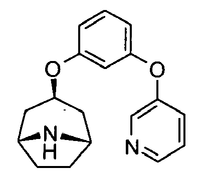

M.S. (ESI) (m/z): 297 [M+H]⁺.

### Procedure III

### EXAMPLE III1.1: 3-exo-(6-Phenylpyridin-2-yloxy)-8-azabicyclo[3.2.]octane

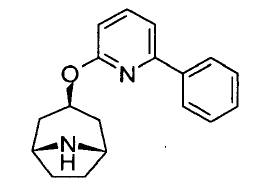

A solution of 6-phenylpyridin-2-ol (75mg, 0.44mmol) in DMF (2.5mL) was added dropwise to a stirred suspension of sodium hydride (21 mg, 0.53mmol) in DMF (0.5ml) in a microwave vessel. The resultant suspension was stirred under a nitrogen atmosphere for 30min. 3-*endo*-methanesulfonyloxy-8-azabicyclo[3.2.1]octane-8-carboxylic acid *tert*-butyl ester (147mg, 0.48mmol) was added to the reaction mixture. The reaction vessel was sealed and then heated at 80°C for 600 seconds then at 100°C for 1200 seconds. The solvent was removed *in vacuo* and the residue partitioned between ethyl acetate and water. The aqueous layer was washed with ethyl acetate and the organics combined, dried over Na₂SO₄ and the solvent evaporated *in vacuo.* The residue was purified by chromatography on silica gel. Elution with DCM with a gradient to 1:99 methanol:DCM afforded a yellow gum. The crude material was dissolved in MeOH and the solution loaded onto a SCX cartridge (500mg). The cartridge was eluted with methanol (15mL) to remove unreacted starting material then with ammonia in methanol (2M, 7.5mL) which was evaporated *in vacuo.* The resultant material was dissolved in DCM (1.5mL) and TFA (0.5mL) added. The reaction mixture was stirred at ambient temperature for 1 h. Volatiles were removed *in vacuo,* the crude material dissolved in methanol (3mL) and the solution loaded onto a SCX cartridge (500mg). The cartridge was eluted with methanol (15mL) to remove impurities. Elution with ammonia in methanol (2M, 7.5mL) followed by evaporation *in vacuo* afforded 3-*exo*-(6-phenylpyridin-2-yloxy)-8-azabicyclo[3.2.1]octane (17.1mg, 0.25mmol; 58%). M.S. (ESI) (m/z): 281 [M+H]⁺.

Similarly prepared were

### EXAMPLE III.2: 3-exo-(2-Phenylpyridin-4-yloxy)-8-azabicyclo[3.2.1]octane

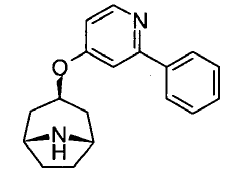

M.S. (ESI) (m/z): 281 [M+H]⁺.

### EXAMPLE III.3: 3-exo-(4-Phenylpyridin-2-yloxy)-8-azabicyclo[3.2.1]octane

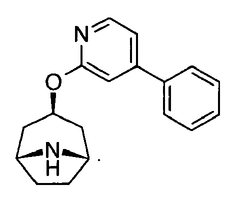

M.S. (ESI) (m/z): 281 [M+H]⁺.

### EXAMPLE III.4: 3-exo-(3-Pyridin-3-ylphenoxy)-8-azabicyclo[3.2.1]octane

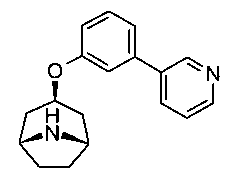

M.S. (ESI) (m/z): 281 [M+H]⁺.

### EXAMPLE III.5: 3-endo-(3-Phenoxyphenoxy)-8-azabicyclo[3.2.1]octane

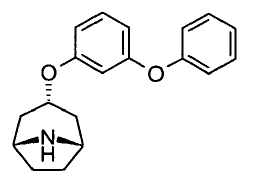

M.S. (ESI) (m/z): 296 [M+H]⁺.

The title compound was prepared from 3-*exo*-methanesulfonyloxy-8-azabicyclo[3.2.1]octane-8-carboxylic acid *tert*-butyl ester

### EXAMPLE III.6: exo 1-{3'-(8-Azabicyclo[3.2.1]oct-3-yloxy)biphenyl-4-yl}ethanone

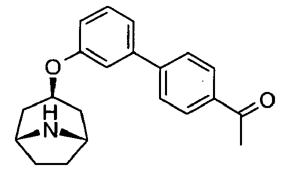

M.S. (ESI) (m/z): 322 [M+H]⁺.

### Procedure IV

### EXAMPLE IV.1: 3-exo-(4'-Trifluoromethylbiphenyl-3-yloxy)-8-azabicyclo[3.2.1]octane

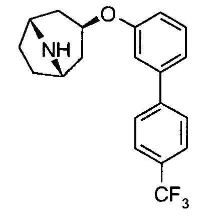

(4,4-Dimethyl-1,1-dioxido-1,2,5-thiadiazolidin-2-yl)triphenylphosphonium (172mg, 0.420mmol) was added to a solution of 3-*endo*-hydroxy-8-azabicyclo[3.2.1]octane-8-carboxylic acid tert-butyl ester (95mg, 0.42mmol), and 4'-trifluoromethylbiphenyl-3-ol (50mg, 0.21 mmol) in THF (2mL). The reaction mixture was stirred for 18h at ambient temperature. Volatiles were removed under reduced pressure. The resultant material was dissolved in DCM (1mL) and TFA (1mL) was added. The reaction mixture was stirred at ambient temperature for 4h. Volatiles were removed under reduced pressure, the crude product dissolved in methanol (2mL) and the solution loaded onto a SCX cartridge (5g).

The cartridge was eluted with methanol (3x10mL) to remove triphenylphosphine oxide. Elution with ammonia in methanol (2M, 10mL) followed by evaporation *in vacuo* afforded crude 3-(4'-trifluoromethylbiphenyl-3-yloxyl-8-azabicyclo[3.2.1 ]octane as an oil. The crude material was then purified by preparative LCMS to afford 3-*exo*-(4'-trifluoromethylbiphenyl-3-yloxy)-8-azabicyclo[3.2.1]octane as the trifluoroacetic acid salt (36.9mg, 0.080mmol, 38%) M.S. (ESI) (m/z): 348 [M+H]⁺.

Similarly prepared were:

### EXAMPLE IV.2: 3-exo-(4-Chlorobiphenyl-3-yloxy)-8-azabicyclo[3.2.1]octane

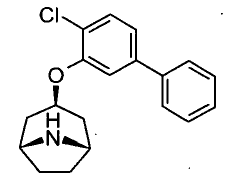

M.S. (ESI) (m/z): 314, 316 [M+H]⁺.

### EXAMPLE IV.3: 3-exo-(2'-Chlorobiphenyl-3-yloxy)-8-azabicyclo[3.2.1]octane

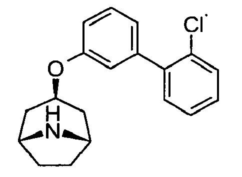

M.S. (ESI) (m/z): 314,'316 [M+H]⁺.

### EXAMPLE IV.4: 3-exo-(3'-Chlorobiphenyl-3-yloxy)-8-azabicyclo[3.2.1]octane

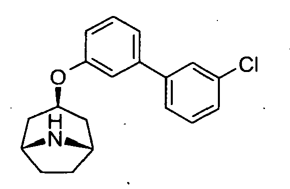

M.S. (ESI) (m/z): 314, 316 [M+H]⁺.

### EXAMPLE IV.5: 3-exo-(4'-Chlorobiphenyl-3-yloxy)-8-azabicyclo[3.2.1]octane

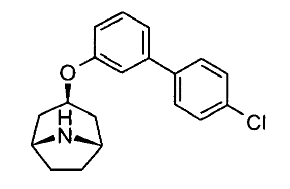

M.S. (ESI) (m/z): 314, 316 [M+H]⁺.

### EXAMPLE IV.6: 3-exo-(2'-Fluorobiphenyl-3-yloxyl)-8-azabicyclo[3.2.1]octane

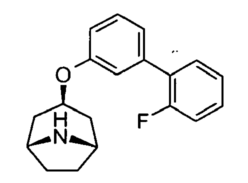

M.S. (ESI) (m/z): 298 [M+H]⁺.

### EXAMPLE IV.7: 3-exo-(4'-Fluorobiphenyl-3-yloxy)-8-azabicyclo[3.2.1]octane

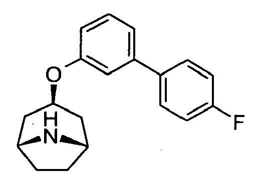

M.S. (ESI) (m/z): 298 [M+H]⁺.

### EXAMPLE IV.8: 3-exo-(2'-Trifluoromethylbiphenyl-3-yloxy)-8-azabicyclo[3.2.1]octane

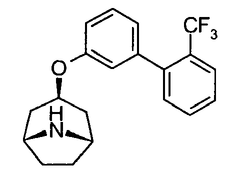

M.S. (ESI) (m/z): 348 [M+H]⁺.

### EXAMPLE IV.9: 3-exo-(2-Fluoro-3-methoxy-5-phenoxyphenoxy)-8-azabicyclo[3.2.1]octane

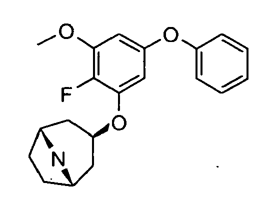

M.S. (ESI) (m/z): 344 [M+H]⁺.

### EXAMPLE IV.10: 3-exo-(3'-Trifluoromethoxybiphenyl-3-yloxy)-8-azabicyclo[3.2.1]octane

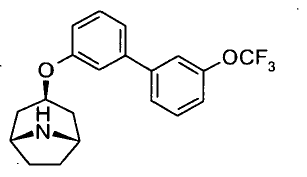

M.S. (ESI) (m/z): 364 [M+H]⁺.

### EXAMPLE IV.11: 3-exo-(2'-Methylbiphenyl-3-yloxy)-8-azabicyclo[3.2.1]octane

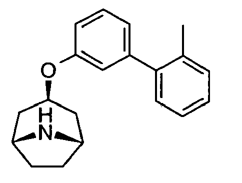

M.S. (ESI) (m/z): 294 [M+H]⁺.

### EXAMPLE IV.12: 3-exo-(3'-Methylbiphenyl-3-yloxy)-8-azabicyclo[3.2.1]octane

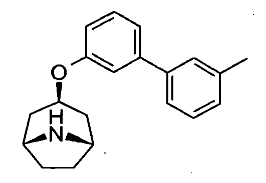

M.S. (ESI) (m/z): 294 [M+H]⁺.

### EXAMPLE IV.13: 3-exo-(3-Phenoxy-4-trifluoromethylphenoxy)-8-azabicyclo[3.2.1]octane

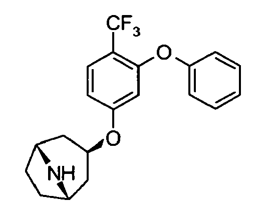

M.S. (ESI) (m/z): 364 [M+H]⁺.

### EXAMPLE IV.14: exo 3'-(8-Azabicyclo[3.2.1]oct-3-yloxy)biphenyl-2-carbonitrile

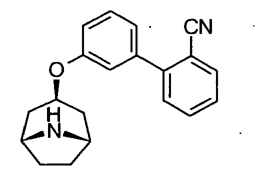

M.S. (ESI) (m/z): 305 [M+H]⁺.

### EXAMPLE IV.15: exo 3'-(8-Azabicyclo[3.2.1]oct-3-yloxy)biphenyl-3-carbonitrile

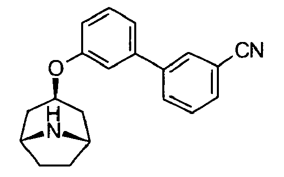

M.S. (ESI) (m/z): 305 [M+H]⁺.

### EXAMPLE IV.16: exo 3'-(8-Azabicyclo[3.2.1]oct-3-yloxy)biphenyl-4-carbonitrile

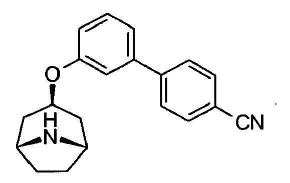

M.S. (ESI) (m/z): 305 [M+H]⁺.

### EXAMPLE IV.17: 3-exo-(3-Phenoxy-5-trifluoromethylphenoxy)-8-azabicyclo[3.2.1]octane

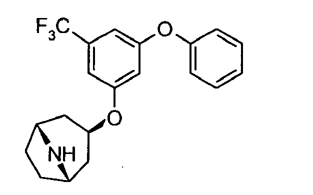

M.S. (ESI) (m/z): 364 [M+H]⁺.

### EXAMPLE IV.18: 3-exo-(4-Methyl-3-phenoxyphenoxy)-8-azabicyclo[3.2.1]octane

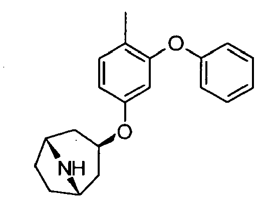

M.S. (ESI) (m/z): 310 [M+H]⁺.

### EXAMPLE IV.19: 3-exo-(3-Chloro-5-phenoxyphenoxy)-8-azabicyclo[3.2.1]octane

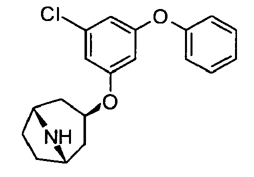

M.S. (ESI) (m/z): 330,332 [M+H]⁺.

### EXAMPLE IV.20: exo 3-(8-Azabicyclo[3.2.1]oct-3 -yloxy)-5-phenoxybenzonitrile

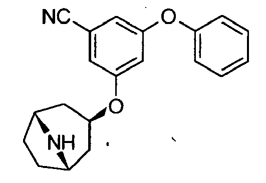

M.S. (ESI) (m/z): 321 [M+H]⁺.

### EXAMPLE IV.21: 3-exo-(3'-Fluorobiphenyl-3-yloy)-8-azabiyclo[3.2.1]octane

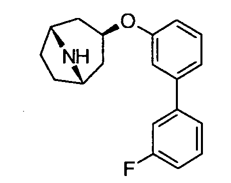

M.S. (ESI) (m/z): 298 [M+H]⁺.

### EXAMPLE IV.22: 3-exo-(3'-Trifluoromethylbiphenyl-3-yloxy)-8-azabiclo[3.2.1]octane

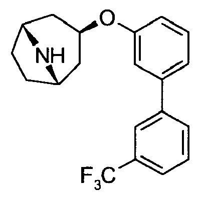

M.S. (ESI) (m/z): 348 [M+H]⁺.

### EXAMPLE IV.23: 3-exo-(4'-Trifluoromethoxybiphenyl-3-yloxy)-8-azabicyclo[3.2.1]octane

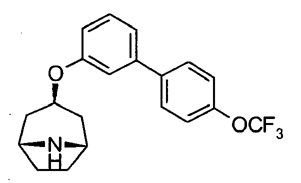

M.S. (ESI) (m/z): 364 [M+H]⁺.

### EXAMPLE IV.24: 3-exo-(2'-Methoxybiphenyl-3-yloxy)-8-azabicyclo[3.2.1]octane

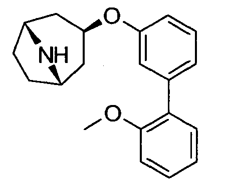

M.S. (ESI) (m/z): 310 [M+H]⁺.

### EXAMPLE IV.25: 3-exo-(3'-Methoxybiphenyl-3-yloxy)-8-azabicyclo[3.2.1]octane

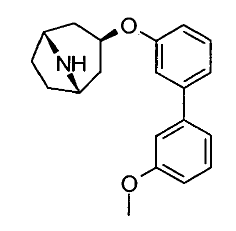

M.S. (ESI) (m/z): 310 [M+H]⁺.

### EXAMPLE IV.26: 3-exo-14'-Methoxybiphenyl-3-yloxy)-8-azabicyclo[3.2.1]octane

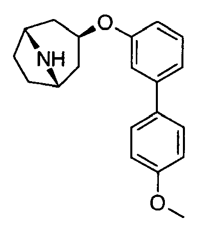

M.S. (ESI) (m/z): 310 [M+H]⁺.

### EXAMPLE IV.27: 3-exo-(5-Phenylpyridin-3-yloxy)-8-azabicyclo[3.2.1]octane

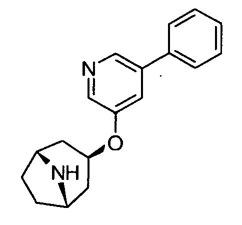

M.S. (ESI) (m/z): 281 [M+H]⁺.

### EXAMPLE IV.28: 3-exo-12-Fluorobiphenyl-3-yloxy)-8-azabicyclo[3.2.1]octane

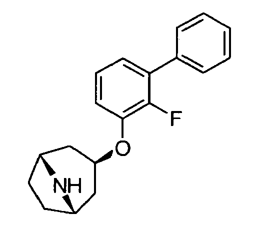

M.S. (ESI) (m/z): 298 [M+H]⁺.

### EXAMPLE IV.29: 3-exo-(4-Methylbiphenyl-3-yloxy)-8-azabicyclo[3.2.1]octane

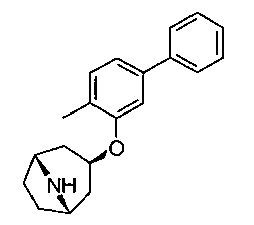

M.S. (ESI) (m/z): 294 [M+H]⁺.

### EXAMPLE IV.30: exo 3'-(8-Azabicyclo[3.2.1]oct-3 -yloxy)-2'-fluorobiphenyl-4-carbonitrile

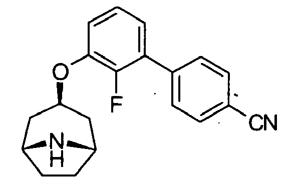

M.S. (ESI) (m/z): 323 [M+H]⁺.

### EXAMPLE IV.31: exo 3-(2'-Trifluoromethoxybiphenyl-3-yloxy)-8-azabicyclo[3.2.1]octane

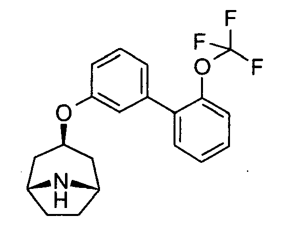

M.S. (ESI) (m/z): 364 [M+H]⁺.

### EXAMPLE IV.32: exo 3'-(8-Azabicyclo[3.2.1]oct-3-yloxy)-biphenyl-3-carbonitrile

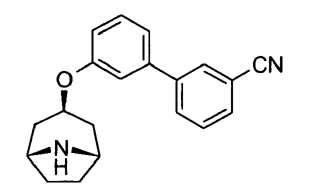

M.S. (ESI) (m/z): 305 [M+H]⁺.

### Procedure V

### EXAMPLE V.1: exo 3'-(8-Methyl-8-azabicyclo[3.2.1]oct-3-yloxy)biphenyl-4-carbonitrile

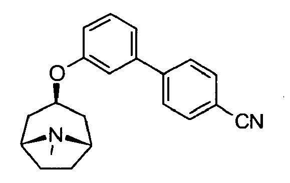

3'-(8-Azabicyclo[3.2.1]oct-3-*exo*-yloxy)biphenyl-4-carbonitrile (10mg, 0.029mmol) was dissolved in methanol (0.5mL) and a solution of 37% formaldehyde in water (11.5µL) was added. Sodium triacetoxyborohydride (12.5mg) was added and the reaction stirred at ambient temperature for 2h. DCM (3mL) and 1 N NaOH (aq) (3mL) were then added to the reaction, the organic and aqueous layers were separated, and the organic layer was concentrated *in vacuo.* The crude product was purified by preparative LCMS to afford 3'-(8-methyl-8-azabicyclo[3.2.1]oct-3-*exo*-yloxy)biphenyl-4-carbonitrile as the trifluoroacetic acid salt (6.4mg, 0.015mmol, 52%) M.S. (ESI) (m/z): 319 [M+H]⁺.

Similarly prepared were:

### EXAMPLE V.2: 3-exo-(3-Chloro-5-phenoxyphenoxy)-8-methyl-8-azabicyclo[3.2.1]octane

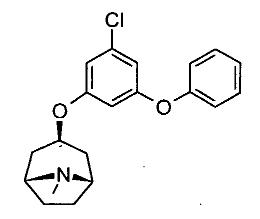

M.S. (ESI) (m/z): 344,346 [M+H]⁺.

### Procedure VI

### EXAMPLE VI.1:3-exo-(6-Methylbiphenyl-3-yloxy)-8-azabicyclo[3.2.1]octane

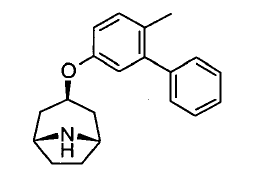

Potassium carbonate (659mg, 4.27mmol), phenylboronic acid (388mg, 3.18mmol), 2-bromo-4-fluorotoluene (0.20mL, 1.59mmol), and tetrakis(triphenylphosphine)palladium(0) (92mg, 5mol%), in DME (3mL) were sealed in a microwave vessel and heated at 80°C for 1200s, then 120°C for 1200s. Ethyl acetate (5mL) was added and the reaction was filtered and concentrated *in vacuo.* The crude material was then purified by flash column chromatography on silica gel. Elution with 10:90 ethyl acetate:heptane afforded 3-fluoro-6-methylbiphenyl (253mg, 1.36mmol, 86%).

A solution of 3-*exo*-hydroxy-8-azabicyclo[3.2.1]octane-8-carboxylic acid *tert*-butyl ester (122mg, 0.563mmol) in DMF (1.5mL) was added to a stirred suspension of 60% sodium hydride in mineral oil (22mg, 0.536mmol) in DMF (0.5mL) under an atmosphere of nitrogen. The reaction was stirred at ambient temperature for 1 h. A solution of 3-fluoro-6-methylbiphenyl (50mg, 0.268mmol) in DMF (0.5mL) was then added and the reaction was heated in a microwave at 180°C for 1200s. The reaction mixture was concentrated *in vacuo*, DCM (1 mL) and TFA (1 mL) were added, and the reaction was stirred for 2h. The crude reaction mixture was loaded onto a SCX cartridge. The cartridge was eluted with methanol (3x10mL) to remove impurities. Elution with ammonia in methanol (2M, 10mL) followed by evaporation *in vacuo* afforded crude 3-*exo*-(6-methylbiphenyl-3-yloxy)-8-azabicyclo[3.2.1]octane. The crude material was then purified by preparative LCMS to afford 3-*exo*-(6-methylbiphenyl-3-yloxy)-8-azabicyclo[3.2.1]octane as the trifluoracetic acid salt (13mg, 0.032mmol, 12%). M.S. (ESI) (m/z): 294 [M+H]⁺.

Similarly prepared were:

### EXAMPLE VI.2: 3-exo-(4-Methylbiphenyl-3-yloxy)-8-azabicyclo[3.2.1]octane

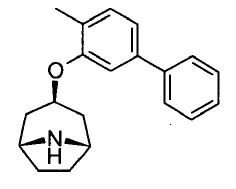

M.S. (ESI) (m/z): 294 [M+H]⁺.

### EXAMPLE VI3: 3-exo-(6-Trifluoromethylbiphenyl-3-yloxy)-8-azabicyclo[3.2.1]octane

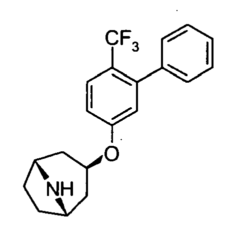

M.S. (ESI) (m/z): 348 [M+H]⁺.

### EXAMPLE VI.4: exo 3-(3-Fluoro-5-pyridin-4-ylphenoxy)-8-azabicyclo[3.2.1]octane

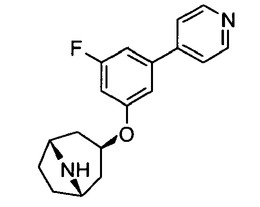

M.S. (ESI) (m/z): 299 [M+H]⁺.

### EXAMPLE VI.5: exo 3-(3-Chloro-5-pyridin-4-ylphenoxy)-8-azabicyclo[3.2.1]octane

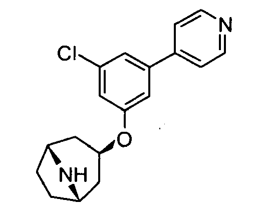

M.S. (ESI) (m/z): 315, 317 [M+H]⁺.

### EXAMPLE VI.6: exo 3-[4-Chloro-3-(pyridin-3-yloxy)phenoxyl-8 azabicyclo[3.2.1]octane

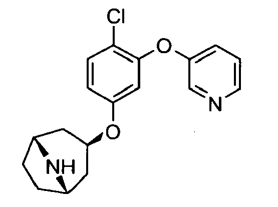

M.S. (ESI) (m/z): 315, 317 [M+H]⁺.

### EXAMPLE VI7: exo 3-(4-Methyl-3-pyridin-4-ylphenoxy)-8-azabicyclo[3.2.1]octane

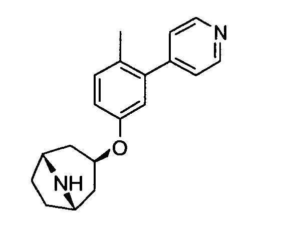

M.S. (ESI) (m/z): 295 [M+H]⁺.

### EXAMPLE VI.8: exo 5-(8-Azabicyclo[3.2.1]oct-3-yloxy)-biphenyl-3,4'-dicarbonitrile

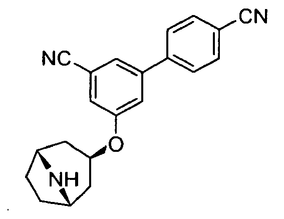

M.S. (ESI) (m/z): 330 [M+H]⁺.

### EXAMPLE VI.9: exo 3'-(8-Azabicyclo[3.2.1]oct-3-yloxy)-5'-chlorobiohenyl-4-carbonitrile

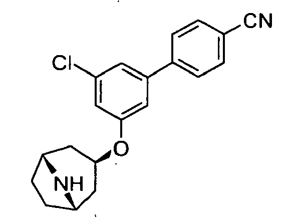

M.S. (ESI) (m/z): 339,341 [M+H]⁺.

### Procedure VII

### EXAMPLE VII1: 3-exo-(4-Chloro-6-phenylpyridin-2-yloxy)-8-azabicyclo[3.2.1]octane

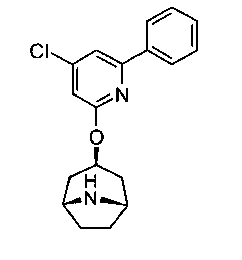

2,4-Dichloro-6-hydroxypyridine (696mg, 4.2mmol), 3-*exo*-hydroxy-8-azabicyclo[3.2.1]octane-8-carboxylic acid *tert*-butyl ester (965mg, 4.2mmol) and (4,4-Dimethyl-1,1-dioxido-1,2,5-thiadiazolidin-2-yl)triphenylphosphonium (4.31g, 10.5mmol) in THF (20mL) were stirred for 16h. Ethyl acetate (50mL) and water (20mL) were added and the organic and aqueous layers were separated. The organic layer was dried over Na₂SO₄ and concentrated *in vacuo*. The crude material was then purified by chromatography on silica gel. Elution with 10:90 ethyl acetate:heptane afforded 3-*exo*-(4,6-dichloropyridin-2-yloxy)-8-azabicyclo[3.2.1]octane-8-carboxylic acid *tert*-butyl ester (1.29g, 3.6mmol, 82%). M.S. (ESI) *m*/*z*: 373,375 [M+H]⁺.

3-*exo*-(4,6-Dichloropyridin-2-yloxy)-8-azabicyclo[3.2.1]octane-8-carboxylic acid *tert*-butyl ester (200mg, 0.54mmol), phenylboronic acid (78mg, 0.64mmol), tetrakis(triphenylphosphine)palladium(0) (31mg, 5mol%) and potassium carbonate (201mg, 1.45mmol) in DME (2.5mL) were heated in the microwave at 100°C for 900s. Ethyl acetate (20mL) and water (10mL) were added and the aqueous and organic layers were separated, dried over Na₂SO₄ and concentrated *in vacuo.* The crude material was then purified by chromatography on silica gel. Elution with 5:95 ethyl acetate:heptane afforded 3-*exo*-(4-chloro-6-phenylpyridin-2-yloxy)-8-azabicyclo[3.2.1]octane-8-carboxylic acid *tert*-butyl ester (68mg, 0.16mmol, 30%). M.S. (ESI) *m*/*z*: 415,417 [M+H]⁺.

3-*exo*-(4-chloro-6-phenylpyridin-2-yloxy)-8-azabicyclo[3.2.1]octane-8-carboxylic acid *tert-*butyl ester (30mg, 0.072mmol) was dissolved in DCM (2mL) and TFA (2mL) and stirred for 30min and then the solvents were removed *in vacuo.* The crude material was then purified by preparative LCMS to afford 3-*exo*-(4-chloro-6-phenylpyridin-2-yloxy)-8-azabicyclo[3.2.1]octane as the trifluoroacetic acid salt (13mg, 0.030mmol, 42%). M.S. (ESI) *m*/*z*: 315,317 [M+H]⁺.

Similarly prepared were:

### EXAMPLE VII.2: 3-exo-(4,6-Diphenylpyridin-2-yloxy)-8-azabicyclo[3.2.1]octane

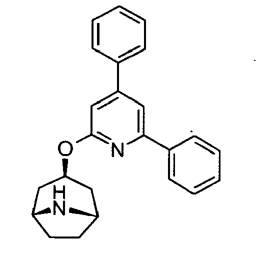

M.S. (ESI) *m*/*z*: 357 [M+H]⁺.

### EXAMPLE VII.3: exo 4-[6-(8-Azabicyclo[3.2.1]oct-3-yloxy)-4-chloropyridin-2-yl]benzonitrile

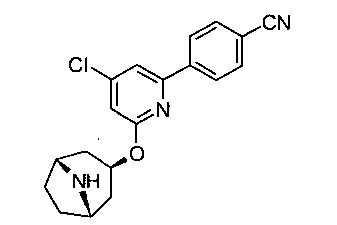

M.S. (ESI) *m*/*z*: 340,342 [M+H]⁺.

### EXAMPLE VII.4: exo 4-[2-(8-Azabicyclo[3.2.1]oct-3-yloxy)-6-chloropyridin-4-yl]benzonitrile

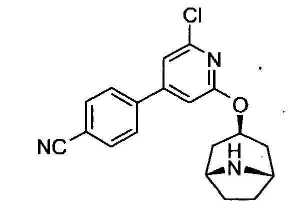

M.S. (ESI) *m*/*z*: 340,342 [M+H]⁺.

### EXAMPLE VII.5: 3-exo-(3-Pyridin-4-ylphenoxy)-8-azabicyclo[3.2.1]octane

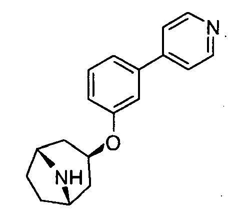

M.S. (ESI) *m*/*z*: 281 [M+H]⁺.

### EXAMPLE VII.6: exo 4-[6-(8-Azabicyclo[3.2.1]oct-3-yloxy)pyridin-2-yl]benzonitrile

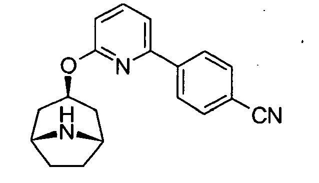

M.S. (ESI) *m*/*z*: 306 [M+H]⁺.

### EXAMPLE VII.7: exo 2-{4-(8-Azabicyclo[3.2.1]oct-3-yloxy)-6-chloro-pyridin-2-yl}benzonitrile

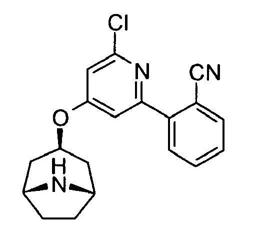

M.S. (ESI) *m*/*z*: 340,342 [M+H]⁺.

### EXAMPLE VII.8 : exo 2-{6-(8-Azabicyclo[3.2.1]oct-3-yloxy)-4-chloropyridin-2-yl}benzonitrile

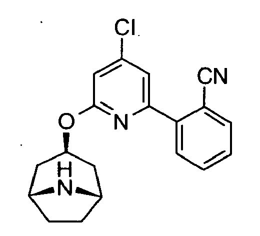

M.S. (ESI) *m*/*z*: 340,342 [M+H]⁺.

### EXAMPLE VII.9: exo 2-{6-(8-Azabicyclo[3.2.1]oct-3-yloxy)-4-chloropyridin-2-yl}benzamide

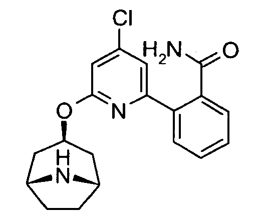

M.S. (ESI) *m*/*z*: 358,360 [M+H]⁺.

### EXAMPLE VII.10: exo -2-[2-(8-Azabicyclo[3.2.1]oct-3-yloxy)-6-chloropyridin-4 yl]benzonitrile

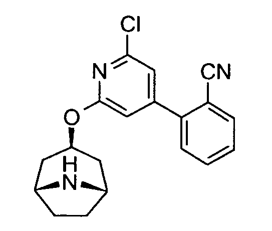

M.S. (ESI) *m*/*z*: 340,342 [M+H]⁺.

### EXAMPLE VII.11: exo 2-[6-(8-Azabicyclo[3.2.1]oct-3-yloxy)pyridin-2-yl]benzonitrile

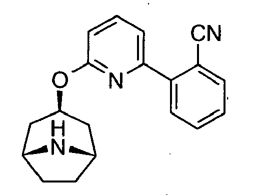

M.S. (ESI) *m*/*z*: 306 [M+H]⁺.

### EXAMPLE VII.12: exo 4-[2-(8-Azabicyclo[3.2.1]oct-3-yloxy)pyridin-4-yl]benzonitrile

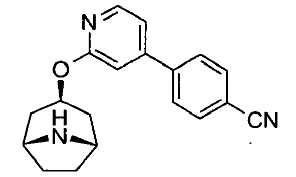

M.S. (ESI) *m*/*z*: 306 [M+H]⁺.

### EXAMPLE VII.13: exo 4-[4-(8-Azabicyclo[3.2.1]oct-3-yloxy)pyridin-2-yl]benzonitrile

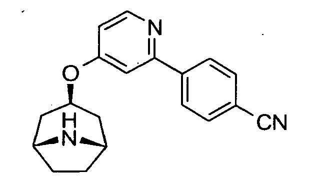

M.S. (ESI) *m*/*z*: 306 [M+H]⁺.

### EXAMPLE VII.14: exo 3-(4,5-Difluorobiphenyl-3-yloxy)-8-azabicyclo[3.2.1]octane

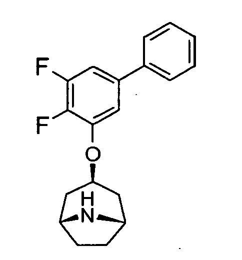

M.S. (ESI) *m*/*z*: 316 [M+H]⁺.

### EXAMPLE VII.15: exo 3-(5-Trifluoromethylbiphenyl-3-yloxy)-8-azabicyclo[3.2.1]octane

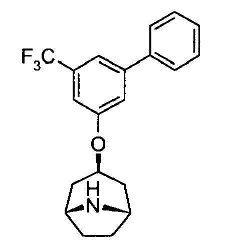

M.S. (ESI) *m*/*z*: 348 [M+H]⁺.

### Procedure VIII

### EXAMPLE VIII.1: 2-(8-Azabicyclo[3.2.1]oct-3-exo-yloxy)-6-phenylisonicotinonitrile

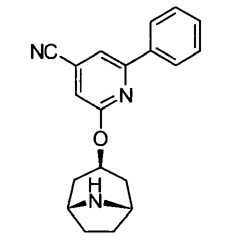

3-*exo*-(4-Chloro-6-pyridin-2-yloxy)-8-azabicyclo[3.2.1]octane-8-carboxylic acid *tert*-butyl ester (30mg, 0.072mmol) prepared as above, zinc(II) cyanide (5mg, 0.043mmol), and tetrakis(triphenylphosphine) palladium(0) (4.2mg, 5mol%) in DMF (0.5mL) were sealed in a microwave vessel and heated at 180°C for 900s. The crude reaction mixture was filtered through a short pad of dicalite. The crude material was then purified by preparative LCMS to afford 3-*exo*-(4-cyano-6-phenylpyridin-2-yloxy)-8-azabicyclo[3.2.1]octane-8-carboxylic acid *tert*-butyl ester (20mg) which was treated directly with DCM (1mL) and TFA (1mL), stirred for 30min and then the solvents were removed *in vacuo.* The crude material was purified by preparative LCMS to afford 2-(8-azabicyclo[3.2.1]oct-3-*exo*-yloxy)-6-phenylisonicotinonitrile as the trifluoroacetic acid salt (4.2mg, 0.010mmol, 14%). M.S. (ESI) *m*/*z*: 306 [M+H]⁺.

Similarly prepared were:

### EXAMPLE VIII.2: exo 2-(8-Azabicyclo[3.2.1]oct-3-yloxy)-6-(4-cyanophenyl)isonicotinonitrile

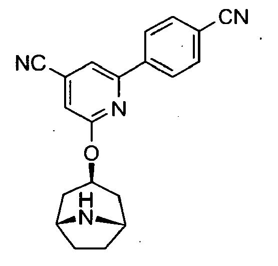

M.S. (ESI) *m*/*z*: 331 [M+H]⁺.

### EXAMPLE VIII.3: exo 2-(8-Azabicyclo[3.2.1]oct-3-yloxy)-6-(2-cyanophenyl)isonicotinonitrile

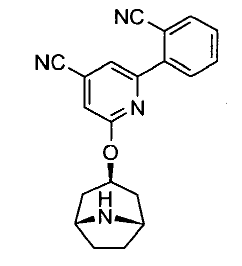

M.S. (ESI) *m*/*z*: 331 [M+H]⁺.

### EXAMPLE VIII.4: exo 2-(8-Azabicyclo[3.2.1]oct-3-yloxy)-6-(2-trifluoromethylphenyl)isonicotinonitrile

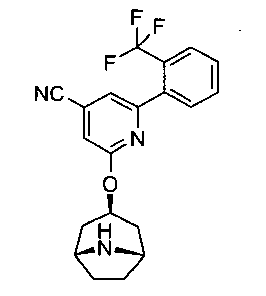

M.S. (ESI) *m*/*z*: 374 [M+H]⁺.

### EXAMPLE VIII.5: exo 2-(8-Azabicyclo[3.2.1]oct-3-yloxy)-6-(2-methoxyphenyl)isonicotinonitrile

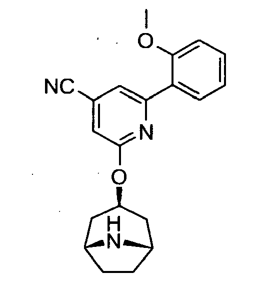

M.S. (ESI) *m*/*z*: 336 [M+H]⁺.

### EXAMPLE VIII.6: exo 2-{6-(8-Azabicyclo[3.2.1]oct-3-yloxy)-4-chloropyridin-2-yl}benzoic acid

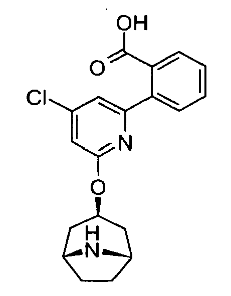

M.S. (ESI) *m*/*z*: 350 [M+H]⁺.

### EXAMPLE VIII.7: exo 2-(8-Azabicyclo[3.2.1]oct-3-yloxy)-6-(3-fluorophenyl)isonicotinonitrile

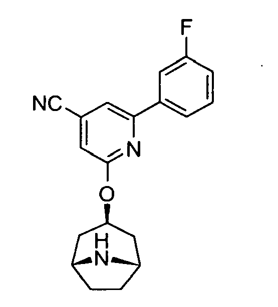

M.S. (ESI) *m*/*z*: 324 [M+H]⁺.

### Procedure IX

### Example IX.1: exo 3-(8-Azabicyclo[3.2.1]oct-3-yloxy)-5-(3-fluoropyridin-2-yl)benzonitrile

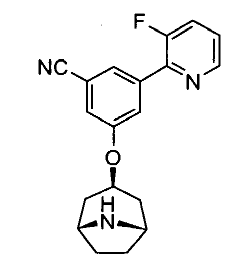

3-Bromo-5-fluorobenzonitrile (8.62g, 43mmol) was dissolved in DMF (20mL). Sodium hydride (2.46g, 62mmol) was added portionwise to the stirred solution under an atmosphere of nitrogen. *Exo tert*-Butyl 3-hydroxy-8-azabicyclo[3.2.1]octane-8-carboxylate (7.0g, 31 mmol) was then added and then reaction was stirred at ambient temperature for 30 minutes. The reaction was quenched with water then the solution was extracted with DCM. The organic layers were combined and dried over MgSO₄. The organic layer was concentrated to leave a brown gum, which was dissolved in DCM (50mL) and washed with water (3x40mL). The organic layer was combined and dried over MgSO₄. The organic layer was concentrated *in vacuo* to leave a brown gum. The crude material was then purified by chromatography on silica gel. Elution with 10:90 ethyl acetate:heptane afforded *exo tert*-butyl 3-(3-bromo-5-cyanophenoxy)-8-azabicyclo[3.2.1]octane-8-carboxylate (7.23g, 17.8mmol, 58%) as a pale yellow solid. *Exo tert*-butyl 3-(3-bromo-5-cyanophenoxy)-8-azabicyclo[3.2.1]octane-8-carboxylate (2.1g, 5.2mmol) in THF (20.6 ml) was treated with triisopropyl borate (3.3 ml, 2.7g, 14mmol,), followed by 1.6M n-butyllithium in hexane (4.0mL, 6.4mmol) over 5 minutes at -78°C, under an atmosphere of nitrogen. The reaction was stirred at -78°C for 2h then warmed to ambient temperature over 60 minutes. The reaction mixture was poured onto 2N HCl (20mL), and extracted with diethyl ether. The combined organic extracts were concentrated *in vacuo* to afford the crude product. The crude product was purified over a short plug of silica gel using 25:75 ethyl acetate:heptane to remove impurities then methanol to afford *exo* 3-[8-(*tert*-butoxycarbonyl)-8-azabicyclo[3.2.1]oct-3-yloxy]-5-cyanophenylboronic acid (1.72 g, 4.6mmol, 90%).

*Exo* 3-[8-(*tert*-butoxycarbonyl)-8-azabicyclo[3.2.1]oct-3-yloxy]-5-cyanophenylboronic acid (80mg, 0.21mmol), 2-chloro-3-fluoropyridine (22mg, 17µL, 0.2mmol), PCy₃ (7.2mg, 0.03mmol), Pd₂(dba)₃ (6.5mg, 7.1µmol), and K₃PO₄ (78mg, 0.37mmol) were placed in a 5 mL microwave vessel followed by the addition of dioxane (2mL) and water (0.5mL). The vessel was then sealed. The reaction was heated at 100°C for 600 seconds. The reaction was quenched by the addition of EtOAc (10mL) and water (5mL) and the aqueous and organic layers were separated. The organic layer was washed with water (2 x 5 mL), then dried over MgSO₄ and concentrated *in vacuo.* The crude material was then purified by chromatography on silica gel. Elution with 1:3 ethyl acetate:heptane afforded exo *tert*-butyl 3-[3-cyano-5-(3-fluoropyridin-2-yl)phenoxy]-8-azabicyclo[3.2.1]octe-8-carboxylate (84mg, 0.20mmol,92%).

*Exo tert-*butyl 3-[3-cyano-5-(3-fluoropyridin-2-yl)phenoxy]-8-azabicyclo[3.2.1]octe-8-carboxylate (84mg, 0.20mmol) was dissolved in DCM (2 mL). TFA (1 mL) was added and the reaction was placed on a mechanical shaker for 1 hour. The solvents were then removed *in vacuo.* The crude reaction mixture was loaded onto a SCX cartridge. The cartridge was eluted with methanol (3x10mL) to remove impurities. Elution with ammonia in methanol (2M, 10mL) followed by evaporation *in vacuo* afforded *exo* 3-(8-azabicyclo[3.2.1]oct-3-yloxy)-5-(3-fluoropyridin-2-yl)benzonitrile (37mg, 0.12mmol, 58%). M.S. (ESI) *m*/*z*: 324 [M+H]⁺.

Similarly prepared were:

### Example IX.2: exo 3-(8-Azabicyclo[3.2.1]oct-3yloxy)-5-(3-chloropyridin-2-yl)benzonitrile

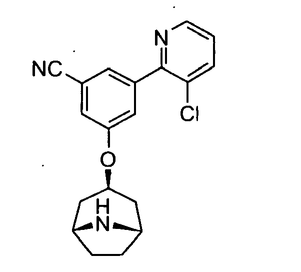

M.S. (ESI) *m*/*z*: 340, 342 [M+H]⁺.

### Example IX.3: exo 6-(3-(8-Azabicyclo[3.2.1]oct-3-yloxy)-5-cyanophenyl)nicotinonitrile

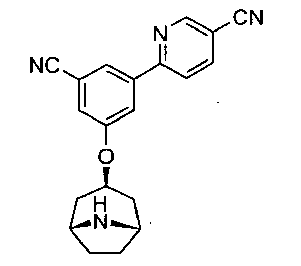

M.S. (ESI) *m*/*z*: 331 [M+H]⁺.

### Example IX.4: exo 3-(8-Azabicyclo[3.2.1]oct-3-yloxy)-5-(3-(trifluoromethyl)pyridin-2-yl)benzonitrile

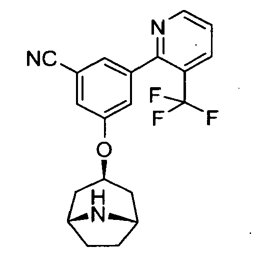

M.S. (ESI) *m*/*z*: 374 [M+H]⁺.

### Example IX.5: exo 3-(8-Azabicyclo[3.2.1]oct-3-yloxy)-5-(pyridin-2-yl)benzonitrile

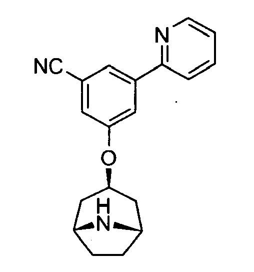

M.S. (ESI) *m*/*z*: 306 [M+H]⁺.

### Example IX.6: exo 3-(8-Azabicyclo[3.2.1]oct-3-yloxy)-5-(3,5-dichloropyridin-2-yl)benzonitrile

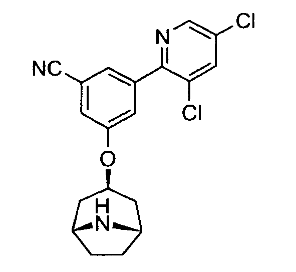

M.S. (ESI) *m*/*z*: 374,376,378 [M+H]⁺.

### Example IX.7: exo 3-(8-Azabicyclo[3.2.1]oct-3-yloxy)-5-(3-methoxypyridin-2-yl)benzonitrile

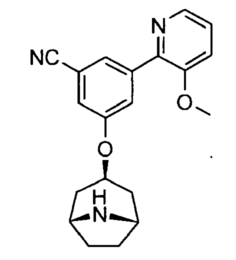

M.S. (ESI) *m*/*z*: 336 [M+H]⁺.

### Example IX.8: exo N-(2-(8-Azabicyclo[3.2.1]oct-3-yloxy)-5-cyanophenyl)pyridin-3-yl)acetamide

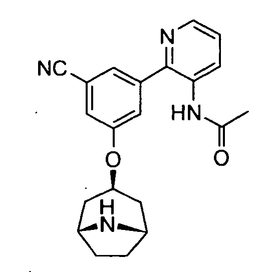

M.S. (ESI) *m*/*z*: 363 [M+H]⁺.

### Example IX.9: exo 3-(8-Azabicyclo[3.2.1]oct-3-yloxy)-5-cyanophenyl)nicotinonitrile

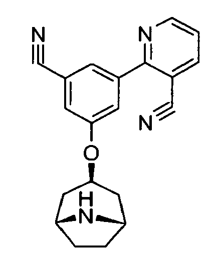

M.S. (ESI) *m*/*z*: 331 [M+H]⁺.

### Example IX.10: exo 3-(8-Azabicyclo[3.2.1]oct-3-yloxy)-5-(pyrimidin-2-yl)benzonitrile

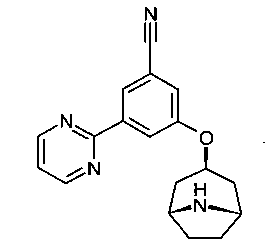

M.S. (ESI) *m*/*z*: 307 [M+H]⁺.

### Example IX.11: exo 3-(8-Azabicyclo[3.2.1]oct-3-yloxy)-5-(pyrimidin-5-yl)benzonitrile

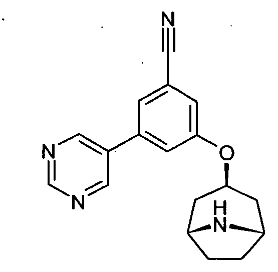

M.S. (ESI) *m*/*z*: 307 [M+H]⁺.

### Example IX.12: exo 3-(8-Azabicyclo[3.2.1]oct-3-yloxy)-5-(isoquinolin-1-yl)benzonitrile

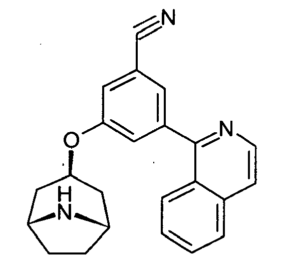

M.S. (ESI) *m*/*z*: 356 [M+H]⁺.

### Procedure X

### Example X.1: 3-exo-(5-Chloro-6-phenoxypyridin-2-yloxy)-8-azabicyclo[3.2.1]octane

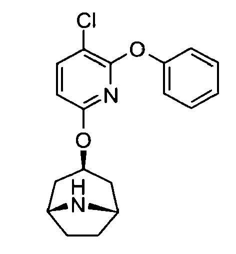

*Exo tert*-butyl 3-(5,6-dichloropyridin-2-yloxy)-8-azabicyclo[3.2.1]octane-8-carboxylate (150mg, 0.4mmol) prepared according to general procedure I was dissolved in DMA (0.5mL) and added to a solution of phenol (56mg, 0.6mmol) and sodium hydride (60% in mineral oil, 15mg, 0.6mmol) in DMA (0.5mL). The reaction was heated to 180°C for 900s using microwave irradiation. Sodium hydride (60% in mineral oil, 10mg, 0.4mmol) was added and the reaction was heated to 180°C for a further 900s using microwave irradiation. The reaction was quenched by the addition of 1 M aqueous NaOH (5mL) and extracted into DCM (10mL). The organic layer was dried over Na₂SO₄ and concentrated *in vacuo*. The crude material was then purified by chromatography on silica gel. Elution with 20:80 ethyl acetate:heptane afforded *exo tert*-butyl 3-(5-chloro-6-phenoxypyridin-2-yloxy)-8-azabicyclo[3.2.1]octane-8-carboxylate (77mg, 0.18mmol, 45%)

*Exo tert*-butyl 3-(5-chloro-6-phenoxypyridin-2-yloxy)-8-azabicyclo[3.2.1 ]octane-8-carboxylate (77mg, 0.18mmol) was dissolved in DCM (2mL) and TFA (1mL) and stirred at ambient temperature for 1 h. Solvents were removed *in vacuo* and the crude material was purified by preparative LCMS to afford 3-*exo*-(5-chloro-6-phenoxypyridin-2-yloxy)-8-azabicyclo[3.2.1]octane (7.1mg, 0.02mmol, 9%). M.S. (ESI) *m*/*z*: 331,333 [M+H]⁺.

Similarly prepared were:

### Example X.2: 3-exo-(6-Phenoxypyridin-2-yloxy)-8-azabicyclo[3.2.1]octane

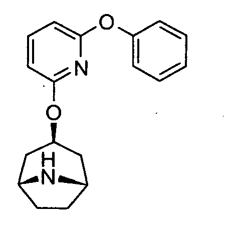

M.S. (ESI) *m*/*z*: 297 [M+H]⁺.

### Procedure XI:

### Assay of monoamine uptake

The *in vitro* test for the inhibition of dopamine and serotonin uptake was performed in Chinese Hamster Ovary cells expressing the human dopamine transporter (hDAT) or the human serotonin transporter (hSERT). The *in vitro* test for the inhibition of noradrenaline uptake was performed in Madin Darby Canine Kidney Cells (MDCK) expressing the human noradrenaline transporter (hNET).

Briefly, cell lines stably overexpressing the appropriate human transporter were propagated and plated according to standard cell culture techniques. Following plating, cells were left to adhere for either one or two days. A 6-point serial dilution (normally 1 E-5M to 1E-10M) of test and reference compounds was prepared, added to the washed cells and incubated for 5 minutes at ambient temperature for dopamine or serotonin transporter overexpressing and 37°C for noradrenaline overexpressing cells. Next, a final concentration of 20 nM of appropriate neurotransmitter (mixture of [³H]-neurotransmitter and non-labelled neurtoransmitter) was added and the cells were incubated for three or five minutes at ambient temperature for dopamine or serotonin transporter overexpressing cells or ten minutes at 37°C for noradrenaline overexpressing cells. Following termination of the assay, Microscint-20 was added directly to the cells and the amount of radioactivity taken up by the cells was estimated by scintillation counting.

pEC₅₀ values indicating inhibition of monoamine uptake were calculated using standard curve fitting techniques.

Table 1 indicates the potency of the representative compounds of the invention.

**Table 1**

| Example | Chemical name | hDAT | hNET | hSERT |
|---|---|---|---|---|
| **IV.19** | *exo* 3-(3-Chloro-5-phenoxyphenoxy)-8-azabicyclo[3.2.1]octane | (+++) | (+++) | (+) |
| **IV.8** | *exo* 3-(2'-Trifluoromethylbiphenyl-3-yloxy)-8-azabicyclo[3.2.1]octane | (+) | (+++) | (++) |
| **IX.3** | *exo* 6-(3-(8-Azabicyclo[3.2.1 ]oct-3-yloxy)-5-cyanophenyl)nicotinonitrile | (+) | (+) | (+++) |
| **1.7** | 3-(5-Fluorobiphenyl-3-yloxy)-8-azabicyclo[3.2.1]octane | (+) | (+++) | (++) |
| **111.5** | 3-*endo*-(3-Phenoxyphenoxy)-8-azabicyclo[3.2.1]octane | (+++) | (++) | (+) |
| **IV.9** | 3-*exo*-(2-Fluoro-3-methoxy-5-phenoxyphenoxy)-8-azabicyclo[3.2.1]octane | (+++) | (+++) | (+) |
| **VI.8** | *exo* 5-(8-Azabicyclo[3.2.1]oct-3-yloxy)-biphenyl-3,4'-dicarbonitrile | (+) | (+) | (+++) |
| **VI.3** | 3-*exo*-(6-Trifluoromethylbiphenyl-3-yloxy)-8-azabicycio[3.2.1]octane | (+) | (+) | (++) |

| | | | | |
|---|---|---|---|---|
| +++ pEC₅₀ >7 ++ pEC₅₀ 6-7 + pEC₅₀ <6 | | | | |

## Claims

1. A 8-azabicyclo[3.2.1]octane derivative of Formula I, 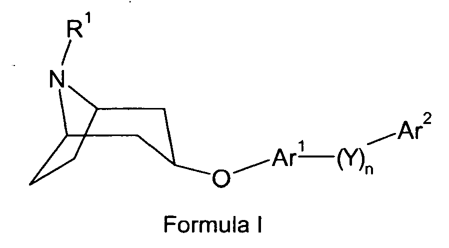 wherein
R¹ is H or C₁₋₅alkyl;
Y is O, S or O(CH₂)ₘ;
m is 1 or 2;
n is 0 or 1;
Ar¹ is phenylene or pyridylene, said phenylene and pyridylene being 1,3-linked with respect to O and when n is 1 with Y and when n is 0 with Ar², said phenylene or pyridylene being optionally substituted with one or two substituents independently selected from halogen, C₁₋₅alkyl, C₁₋₅alkoxy, C₃₋₆cycloalkyl, C₂₋₅alkenyl, C₂₋₅alkynyl, phenyl, CN and hydroxy, wherein said C₁₋₅alkyl and C₁₋₅alkoxy are optionally substituted with one to three halogens and wherein the oxygen of said hydroxy is optionally bonded to Ar² to form a 5-membered ring;
Ar² is phenyl or a 5-6 membered heteroaryl, said phenyl or 5-6 membered heteroaryl being optionally substituted with one to three substituents independently selected from halogen, C₁₋₅alkyl, C₁₋₅alkoxy, CN, CONR²R³, CO₂R⁴ NHCOR⁵ and hydroxy, wherein said C₁₋₅alkyl and C₁₋₅alkoxy are optionally substituted with one to three halogens and wherein the oxygen of said hydroxy is optionally bonded to Ar¹ to form a 5-membered ring;
R²-R⁴ are independently H or C₁₋₅alkyl and
R⁵ is C₁₋₅alkyl
or a pharmaceutically acceptable salt or solvate thereof.

2. The 8-azabicyclo[3.2.1]octane derivative according to claim 1 wherein R¹ is H or methyl.

3. The 8-azabicyclo[3.2.1]octane derivative according to any one of claims 1-2, wherein n is 0.

4. The 8-azabicyclo[3.2.1]octane derivative according to any one of claims 1-2, wherein Y is O and n is 1.

5. The 8-azabicyclo[3.2.1]octane derivative according to any one of claims 1-4, wherein Ar¹ is phenyl or pyridyl optionally substituted with 1-2 substituents selected from chloro, fluoro, methyl, methoxy or CN.

6. The 8-azabicyclo[3.2.1]octane derivative according to any one of claims 1-5, wherein Ar² is phenyl or pyridyl optionally substituted with 1-2 substituents selected from chloro, fluoro, methyl, methoxy, CN or CF₃.

7. A 8-azabicyclo[3.2.1]octane derivative selected from:
3-*exo*-(5-chlorobiphenyl-3-yloxy)-8-azabicyclo[3.2.1]octane;
*exo* 5-(8-azabicyclo[3.2.1]oct-3-yloxy)biphenyl-3-carbonitrile;
*exo* 3-(8-azabicyclo[3.2.1]oct-3-yloxy)-5-phenoxybenzonitrile;
3-*exo*-(4'-methoxybiphenyl-3-yloxy)-8-azabicyclo[3.2.1]octane;
*exo* 3'-(8-azabicyclo[3.2.1]oct-3 -yloxy)biphenyl-4-carbonitrile;
3-*exo*-(3-pyridin-4-ylphenoxy)-8-azabicyclo[3.2.1]octane;
*exo* 2-{6-(8-azabicyclo[3.2.1]oct-3-yloxy)-4-chloropyridin-2-yl]benzonitrile;
*exo* 2-(8-azabicyclo[3.2.1]oct-3-yloxy}-6-(2-cyanophenyl)isonicotinonitrile;
*exo* 3-[(8-azabicyclo[3.2.1]oct-3-yl)oxy]-5-(3-chloropyridin-2-yl)benzonitrile;
*exo* 3-(8-azabicyclo[3.2.1]oct-3-yloxy)-5-cyanophenyl)nicotinonitrile;
*exo* 3-(8-azabicyclo[3.2.1]oct-3-yloxy)-5-(3-fluoropyridin-2-yl)benzonitrile;
*exo* 3'-(8-azabicyclo[3.2.1]oct-3-yloxy)biphenyl-2-carbonitrile;
*exo* 2-[6-(8-azabicyclo[3.2.1]oct-3-yloxy)pyridin-2-yl]benzonitrile;
3'-(8-azabicyclo[3.2.1]oct-3-*exo*-yloxy)-2'-fluorobiphenyl-4-carbonitrile and
2-(8-azabicyclo[3.2.1]oct-3-*exo*-yloxy)-6-phenylisonicotinonitrile
or a pharmaceutically acceptable salt or solvate thereof.

8. A 8-azabicyclo[3.2.1]octane derivative according to any one of claims 1-7 for use in therapy.

9. A pharmaceutical composition comprising a 8-azabicyclo[3.2.1]octane derivative according to any one of claims 1-7 in admixture with one or more pharmaceutically acceptable auxiliaries.

10. Use of a 8-azabicyclo[3.2.1]octane derivative according to any one of claims 1-7 for the manufacture of a medicament for the treatment or prevention of a disease or disorder which is responsive to monoamine neurotransmitter reuptake in the nervous system.

11. Use according to claim 10, wherein the medicament is for the treatment or prevention of depression or pain.

## Patentansprüche

1. 8-Azabicyclo[3.2.1]octan-Derivat gemäss Formel I, 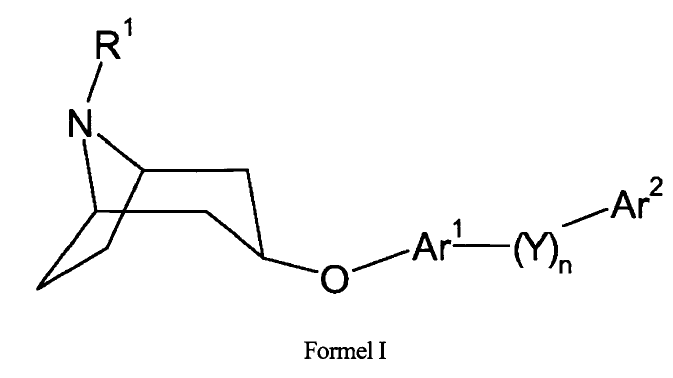 wobei
R¹ H oder C₁₋₅Alkyl ist;
Y O, S oder O(CH₂)ₘ ist;
m 1 oder 2 ist;
n 0 oder 1 ist;
Ar¹ Phenylen oder Pyridylen ist, wobei besagtes Phenylen und Pyridylen 1,3-verbunden in Bezug auf O ist und, wenn n 1 ist, mit Y, und wenn n 0 ist, mit Ar², wobei besagtes Phenylen oder Pyridylen optional substitutiert ist mit einem oder zwei Substituenten, die unabhängig ausgewählt sind aus einem Halogen, C₁₋₅Alkyl, C₁₋₅Alkoxy, C₃₋₆Cycloalkyl, C₂₋₅Alkenyl, C₂₋₅Alkynyl, Phenyl, CN und Hydroxy, wobei besagtes C₁₋₅Alkyl und C₁₋₅Alkoxy optional substitutiert sind mit einem bis drei Halogenen und wobei der Sauerstoff des besagten Hydroxy optional verbunden ist mit Ar², um einen 5-elementigen Ring auszubilden;
Ar² Phenyl oder ein 5-6 elementiges Heteroaryl ist, wobei besagtes Phenyl oder 5-6 elementiges Heteroaryl optional substitutiert sind mit einem bis drei Substituenten, die unabhängig ausgewählt sind aus einem Halogen, C₁₋₅Akyl, C₁₋₅Alkoxy, CN, CONR²R³, CO₂R⁴, NHCOR⁵ und Hydroxy,
wobei besagtes C₁₋₅Alkyl und C₁₋₅Alkoxy optional substitutiert sind mit einem bis drei Halogenen und wobei der Sauerstoff des besagten Hydroxy optional verbunden ist mit Ar¹, um einen 5-elementigen Ring auszubilden;
R²-R⁴ unabhängig H oder C₁₋₅Alkyl sind und
R₅ C₁₋₅Alkyl ist
oder ein pharmazeutisch verträgliches Salz oder Solvat davon.

2. 8-Azabicyclo[3.2.1]octan-Derivat gemäss Anspruch 1, wobei R¹ H oder Methyl ist.

3. 8-Azabicyclo[3.2. 1]octan-Derivat gemäss irgendeinem der Ansprüche 1 bis 2, wobei n O ist.

4. 8-Azabicyclo[3.2.1]octan-Derivat gemäss irgendeinem der Ansprüche 1 bis 2, wobei Y O ist und n 1 ist.

5. 8-Azabicyclo[3.2.1]octan-Derivat gemäss irgendeinem der Ansprüche 1 bis 4, wobei Ar¹ Phenyl oder Pyridyl ist, optional substitutiert mit 1-2 Substituenten, die ausgewählt sind aus Chlor, Fluor, Methyl, Methoxy oder CN.

6. 8-Azabicyclo[3.2.1]octan-Derivat gemäss irgendeinem der Ansprüche 1 bis 5, wobei Ar² Phenyl oder Pyridyl ist, optional substitutiert mit 1-2 Substituenten, die ausgewählt sind aus Chlor, Fluor, Methyl, Methoxy, CN oder CF₃.

7. 8-Azabicyclo[3.2.1]octan-Derivat ausgewählt aus:
3-*exo*-(5-Chlorbiphenyl-3-yloxy)-8-azabicyclo[3.2.1]octan;
*exo*-5-(8-Azabicyclo[3.2.1]oct-3-yloxy)biphenyl-3-carbonitril;
*exo* 3-(8-Azabicyclo[3.2.1]oct-3-yloxy)-5-phenoxybenzonitril;
3-*exo*-(4'-methoxybiphenyl-S-yloxy-[delta]-azabicyclo[beta]octan;
*exo*-3'-(8-Azabicyclo[3.2.1]oct-3-yloxy)biphenyl-4-carbonitril;
3-*exo*-(3-Pyridin-4-ylphenoxy)-8-Azabicyclo[3.2.1]octane;
*exo*-2-{6-(8-Azabicyclo[3.2.1]oct-3-yloxy)-4-Chlorpyridin-2-yl]benzonitril;
*exo*-2-(8-Azabicyclo[3.2.1]oct-3-yloxy)-6-(2-cyanophenyl)isonicotinonitril;
*exo*-3-[(8-Azabicyclo[3.2.1]oct-3-yl)oxy]-5-(3-Chlorpyridin-2-yl)benzonitril;
*exo*-3-(8-Azabicyclo[3.2.1]oct-3-yloxy)-5-cyanophenyl)nicotinonitril;
*exo*-3-(8-Azabicyclo[3.2.1]oct-3-yloxy)-5-(3-fluorpyridin-2-yl)benzonitril;
*exo*-3'-(8-Azabicyclo[3.2.1]oct-3-yloxy)biphenyl-2-carbonitril;
*exo*-2-[6-(8-Azabicyclo[3.2.1]oct-3-yloxy)pyridin-2-yl]benzonitril;
3-(8-Azabicyclo[3.2.1]oct-3-*exo*-yloxy)-2'-fluorbiphenyl-4-carbonitril; und
2-(8-Azabicyclo[3.2.1]oct-3-*exo*-yloxy)-6-phenylisonicotinonitril
oder ein pharmazeutisch verträgliches Salz oder Solvat davon.

8. 8-Azabicyclo[3.2.1]octan-Derivat gemäss irgendeinem der Ansprüche 1 bis 7 für die Verwendung in der Therrapie.

9. Pharmazeutische Zusammensetzung, umfassend ein 8-Azabicyclo[3.2.1]octan-Derivat gemäss irgendeinem der Ansprüche 1 bis 7 in Beimischung mit einem oder mehreren pharmazeutisch verträglichen Hilfsstoffen.

10. Verwendung eines 8-Azabicyclo[3.2.1]octan-Derivats gemäss irgendeinem der Ansprüche 1 bis 7 für die Herstellung eines Medikamentes für die Behandlung oder Vorsorge einer Krankheit oder Störung, die auf Monoamin-Neurotransmitter-Wiederaufnahme in das Nervensystem empfindlich ist.

11. Verwendung gemäss Anspruch 10, wobei das Medikament für die Behandlung oder Vorsorge von Depression oder Schmerz ist.

## Revendications

1. Un dérivé 8-azabicyclo[3.2.1]octane de formule I, 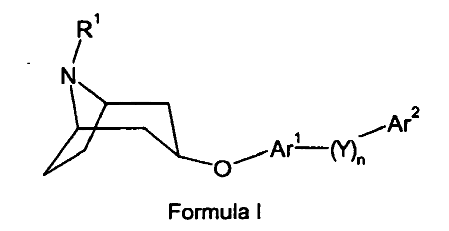 dans lequel
- R¹ est H ou C₁₋₅alkyl;
- Y est O, S ou O(CH₂)ₘ;
- m est 1 ou 2;
- n est 0 ou 1;
- Ar¹ est le phénylène ou pyridylène, lesdits phénylène et pyridylène étant -1,3 liés par rapport à O et quand n est 1 avec Y et quand n est 0 avec Ar², ledit phénylène ou pyridylène étant éventuellement substitués par un ou deux substituants indépendamment sélectionné parmi l'halogène, C₁₋₅alkyl, C₁₋₅alkoxy, C₃₋₆cycloalkyl, C₂₋₅alkenyl, C₂₋₅alkynyl, phényl, CN et hydroxy, dans lequel lesdits C₁₋₅alkyl et C₁₋₅alkoxy sont éventuellement substitués par un à trois halogènes et dans lequel l'oxygène dudit hydroxy est éventuellement lié à Ar² pour former un cycle à 5 atomes;
- Ar² est un radical phényl ou un groupe hétéroaryl à 5-6 chaînons, ledit radical phényl ou hétéroaryl à 5-6 atomes étant éventuellement substitué par un à trois substituants indépendamment sélectionné parmi l'halogène, C₁₋₅alkyl, C₁₋₅alkoxy, CN, CONR²R³, CO₂R⁴, NHCOR⁵ et hydroxy, dans lequel lesdits radicaux C₁₋₅alkyl et C₁₋₅alkoxy sont éventuellement substitués par un à trois halogènes et dans lequel l'oxygène dudit hydroxy est éventuellement lié à Ar¹ pour former un cycle à 5 atomes;
- R²-R⁴ sont, indépendamment H ou C₁₋₅alkyl et
- R⁵ est C₁₋₅alkyl
ou un sel pharmaceutiquement acceptable ou solvate en dérivant.

2. Le dérivé 8-azabicyclo[3.2.1]octane selon la revendication 1, dans lequel R¹ est H ou un groupe méthyl.

3. Le dérivé 8-azabicyclo[3.2.1]octane selon l'une quelconque des revendications 1-2, dans lequel n est 0.

4. Le dérivé 8-azabicyclo[3.2.1]octane selon l'une quelconque des revendications 1-2, dans lequel Y est O et n est 1.

5. Le dérivé 8-azabicyclo[3.2.1]octane selon l'une quelconque des revendications 1-4, dans lequel Ar¹ est un radical phényl ou pyridyl éventuellement substitué par 1-2 substituants choisis parmi le chlore, fluore, méthyl, méthoxy ou CN.

6. Le dérivé 8-azabicyclo[3.2.1]octane selon l'une quelconque des revendications 1-5, dans lequel Ar² est un radical phényl ou pyridyl éventuellement substitué par 1-2 substituants choisis parmi le chlore, fluore, méthyl, méthoxy, CN ou CF₃.

7. Un dérivé 8-azabicyclo[3.2.1]octane choisi parmi :
3-*exo*-(5-chlorobiphényl-3-yloxy)-8-azabicyclo[3.2.1]octane;
*exo* 5-(8-azabicyclo[3.2.1]oct-3-yloxy)-biphényl-3-carbonitrile;
*exo* 3-(8-azabicyclo[3.2.1]oct-3-yloxy)-5-phenoxybenzonitrile;
3-*exo*-(4'-méthoxybiphényl-3-yloxy)-8-azabicyclo[3.2.1]octane;
*exo* 3'-(8-azabicyclo[3.2.1]oct-3-yloxy)-biphényl-4-carbonitrile;
3-*exo*-(3-pyridin-4-ylphenoxy)-8-azabicyclo[3.2.1]octane;
*exo* 2-{6-(8-azabicyclo[3.2.1]oct-3-yloxy)-4-chloropyridin-2-yl}benzonitrile;
*exo* 2-(8-azabicyclo[3.2.1]oct-3-yloxy)-6-(2-cyanophényl)-isonicotinonitrile;
*exo* 3-[(8-azabicyclo[3.2.1]oct-3-yl)-oxy]-5-(3-chloropyridin-2-yl)-benzonitrile;
*exo* 3-(8-azabicyclo[3.2.1]oct-3-yloxy)-5-cyanophényl)-nicotinonitrile;
*exo* 3-(8-azabicyclo[3.2.1]oct-3-yloxy)-5-(3-fluoropyridin-2-yl)-benzonitrile;
*exo* 3'-(8-azabicyclo[3.2.1]oct-3-yloxy)biphényl-2-carbonitrile;
*exo* 2-[6-(8-azabicyclo[3.2.1]oct-3-yloxy)pyridin-2-yl]benzonitrile;
3'-(8-azabicyclo[3.2.1]oct-3-exo-yloxy)-2'-fluorobiphényl-4-carbonitrile et
2-(8-azabicyclo[3.2.1]oct-3-exo-yloxy)-6-phénylisonicotinonitrile
ou un sel pharmaceutiquement acceptable ou solvate en dérivant.

8. Un dérivé 8-azabicyclo[3.2.1]octane selon l'une quelconque des revendications 1-7 pour un usage thérapeutique.

9. Une composition pharmaceutique comprenant un dérivé 8-azabicyclo[3.2.1]octane selon l'une quelconque des revendications 1-7 en mélange avec un ou plusieurs adjuvants pharmaceutiquement acceptables.

10. Utilisation d'un dérivé 8-azabicyclo[3.2.1]octane selon l'une quelconque des revendications 1-7 pour la fabrication d'un médicament pour le traitement ou la prévention d'une maladie ou d'une affection qui est sensible à la recapture d'un neurotransmetteur monoamine par le système nerveux.

11. Utilisation selon la revendication 10, dans laquelle le médicament est destiné au traitement ou la prévention de la dépression ou de la douleur.
